# EUROPEAN PATENT SPECIFICATION

(11) **EP 3 178 501 B1**
(45) Date of publication and mention of the grant of the patent: **01.05.2019**
(21) Application number: 16198801.9
(22) Date of filing: 02.03.2012
(51) Int. Cl.: A61L 29/08, A61L 29/16, A61M 25/10, A61M 29/02

(54) **ELUTING MEDICAL DEVICES**
ELUIERENDE MEDIZINISCHE VORRICHTUNGEN
DISPOSITIFS MÉDICAUX D'ÉLUTION

(30) Priority: 04.03.2011 US 201161449427 P; 16.11.2011 US 201161560659 P; 01.03.2012 US 201213409843
(43) Date of publication of application: 14.06.2017
(62) Divisional of application: 12709752.5
(73) Proprietor: W.L. Gore & Associates, Inc., Newark DE 19714 (US)
(72) Inventor: CAMPBELL, Carey, V., Flagstaff, Arizona 86004 (US); CLEEK, Robert, L., Flagstaff, Arizona 86004 (US); HOLLAND, Theresa, A., Flagstaff, Arizona 86001 (US); KRANZLER, Thane, L., Flagstaff, Arizona 86001 (US); LI, Mei, Flagstaff, Arizona 86001 (US); TRAPP, Benjamin, M., Flagstaff, Arizona 86001 (US)
(74) Representative: HGF Limited

(56) References cited:
- WO-A1-03/015677
- WO-A2-2008/064058
- US-A1- 2008 015 500

## Description

### BACKGROUND

The systemic administration of therapeutic agents treats the body as a whole even though the disease to be treated may be localized. In some cases of localized disease, systemic administration may not be desirable because the drug agents may have unwanted effects on parts of the body which are not to be treated or because treatment of the diseased part of the body requires a high concentration of a drug agent that may not be achievable by systemic administration.

It is therefore often desirable to administer therapeutic agents to only localized sites within the body. Common examples of where this is needed include cases of localized disease (*e.g.,* coronary heart disease) and occlusions, lesions, or other disease in body lumens. Several devices and methods for localized drug delivery are known. In one example, such devices are drug delivery balloons, and methods of their use include the steps of coating a balloon attached to a balloon catheter with a drug and a carrier matrix, inserting the catheter into a blood vessel, tracking the balloon to a desired location, and expanding the balloon against the surrounding tissue to transfer the drug locally at the intended treatment site.

One of the potential drawbacks to localized drug delivery is the possibility of premature or unintended release of the drug, the carrier matrix, and/or the drug/carrier matrix combination. This may occur during tracking and placement at the treatment site of a drug delivery device and post delivery as the device is withdrawn from the body. Such unintended release may result from drug diffusion, device contact with areas proximate the treatment site, or washing of the drug from the surface of the delivery device due to blood flow. This is of particular concern when the device comprises a therapeutic agent of a type or dosage not intended to be released to tissue or blood outside the treatment site.

Drugs or coating components shed in this unwanted fashion may be in particulate form or may be in solution. The release of particles is known as "particulation". Particulation of large particles can create problems such as ischemia in tissues, especially in tissues supplied by small diameter vessels. Furthermore, the resulting effects of biodistribution of such particles are not well understood and may result in adverse effects.

When combining a drug with an implantable device, the drug may be in a solid form (as a particulate or crystal) but is preferably released from the device as a solubilized molecule. The advantages of localized, solubilized drug delivery are believed to be uniform drug distribution at the treatment site, well-known drug biodistribution, and the avoidance of particulation.

WO03/015677 discloses expandable medical devices comprising a hydrophilic coating, a therapeutic agent and a sheath which has an open microstructure of nodes/fibrils that allows the transfer of the therapeutic agent to an external area. In view of the potential drawbacks to current, localized drug delivery, there exists a need for devices and methods that allow for controlled, localized delivery of drug agents, especially soluble agents, to specific treatment sites within a mammalian body that avoids particulation and premature or unintended drug release away from the intended treatment site, while ensuring that desired dosing occurs.

### SUMMARY

The invention is directed to an expandable balloon catheter that delivers a therapeutic agent to a vessel or other lumen of cavity that enables consistent "on-demand" delivery of the agent, while not substantially eluting or releasing said therapeutic agent as the device is being tracked to the desired treatment site. The balloon catheter of the current invention comprises an expandable member with or without a structural layer serving as a substrate over said expandable member, at least one hydrophilic coating comprising at least one therapeutic agent on the expandable member or structural layer, and an outer sheath comprising a variably permeable microstructure. During use, the underlying hydrophilic coating becomes hydrated or partially hydrated and facilitates fluid transfer across the outer sheath. However, said outer sheath's closed microstructure in the unexpanded state prevents unwanted, premature release of said therapeutic agent. Upon expansion, the outer sheath disposed over the expandable member or structural layer transforms from a closed microstructure to an open microstructure allowing the hydrated or partially hydrated coating and said therapeutic agent to be transferred (*e.g.* pushed) outward. Once the hydrated or partially hydrated hydrophilic coating passes through the sheath, the therapeutic agent is delivered to the treatment site. In another embodiment, the hydrated or partially hydrated coating comprises a soluble therapeutic agent and once the outer sheath is expanded, the therapeutic agent is transferred through the sheath. In another embodiment, said expandable member is a medical balloon.

In another embodiment, the invention comprises a balloon catheter comprising an expandable member, a coating comprising a therapeutic agent disposed around said expandable member, a sheath disposed around said coating, wherein said sheath has a variably permeable microstructure that initially prevents or limits unintended transfer of therapeutic agent through said sheath, wherein said coating and therapeutic agent are disposed between the surface of the expandable member and the sheath, and wherein when said expandable member and sheath are expanded, said sheath allows rapid transfer of said coating and therapeutic agent to an area external to said sheath when said sheath is in an unexpanded state while preventing transfer of particles out of said sheath greater than about 25 microns in size. Said sheath allows rapid transfer of said coating and therapeutic agent because said sheath rapidly wets out during expansion. In another embodiment, said sheath undergoes only microscopic wetting in a vessel while said balloon and sheath are in the unexpanded state and being tracked to a desired location within a vessel. In another embodiment, bodily fluids substantially wet-out the sheath when said sheath is expanded. In another embodiment, said sheath is modified to include a hydrophilic component located within at least a part of the sheath and/or on part or all of said sheath's external surface. In another embodiment, said hydrophilic component of said sheath also wets the sheath before and as said sheath is expanded. In another embodiment, substantially all of said sheath is wet by the time said sheath is fully expanded (*i.e.,* expanded to its rated or nominal diameter). In another embodiment, fluid external to said sheath is allowed to flow through said sheath and contact said therapeutic agent before and as said sheath is expanded. In another embodiment, said wetting of the sheath is facilitated when said sheath is in contact with the vessel wall. In another embodiment, said sheath comprises a fluoropolymer. In another embodiment, the outer sheath is wet-out by a prescribed preparatory procedure prior to being inserted into the patient. Said sheath comprises a microstructure comprised of nodes interconnected by fibrils. In another embodiment, said nodes are aligned longitudinally to the longitudinal axis of said balloon catheter and said fibrils are aligned circumferentially to said axis. In another embodiment, said nodes are aligned circumferentially to the longitudinal axis of said balloon catheter and said fibrils are aligned longitudinally to said axis. In another embodiment, the distance between said fibrils increases as said outer sheath expands. In another embodiment, the distance between said nodes increases as said outer sheath expands. In another embodiment, the orientation of said nodes and/or fibrils changes as said outer sheath expands. In another embodiment, said sheath comprises expanded polymers, such as polytetrafluoroethylene (ePTFE). In another embodiment, said coating comprises a hydrophilic component. In another embodiment, said therapeutic agent is a hydrophilic agent. In another embodiment, said coating comprises at least one compound selected from the group consisting of benzethonium chloride, poloxamer-188, polyethylene glycol, sodium salicylate, and hydroxypropyl-β-cyclodextrin. In another embodiment, said therapeutic agent is a hydrophobic agent. In another embodiment, said therapeutic agent is paclitaxel. In another embodiment, said expandable member further comprises a structural layer. In another embodiment, said structural layer comprises said coating and therapeutic agent. The microstructure of the outer sheath changes as said expandable member expands.

The invention comprises a balloon catheter comprising, a balloon comprising a coating and a therapeutic agent disposed around the outer surface of said balloon, a sheath disposed around said balloon wherein said sheath has a microstructure composed of nodes interconnected by fibrils and has characteristics which prevent macroscopic wetting of said sheath in the unexpanded state, wherein said coating and therapeutic agent are disposed between the surface of the balloon and the sheath, and wherein when said balloon and sheath are expanded, substantially all of said sheath wets out rapidly and allows rapid transfer of said coating through the outer sheath. In one embodiment, said coating is transferred through said outer sheath and onto or into a target tissue. In one embodiment, upon expansion said coating is transferred through said outer sheath in a hydrated or partially hydrated state. In another embodiment, said coating remains substantially adhered to the target tissue for greater than 1 minute after contact between balloon and treatment site is substantially eliminated. In another embodiment, said sheath undergoes microscopic wetting in a vessel while said balloon and sheath are in the unexpanded state and being delivered to a desired location within a vessel. In another embodiment, bodily fluids substantially wet-out the sheath when said sheath is expanded. In another embodiment, said coating also wets the sheath when said sheath is expanded. In another embodiment, substantially all of said sheath is wet by the time said sheath is fully expanded. In another embodiment, said wetting of the sheath is facilitated when said sheath is in contact with a vessel wall. In another embodiment, said sheath comprises a fluoropolymer. In another embodiment, said nodes are aligned longitudinally to the longitudinal axis of said balloon catheter and said fibrils are aligned circumferentially to said axis. In another embodiment, said nodes are aligned circumferentially to the longitudinal axis of said balloon catheter and said fibrils are aligned longitudinally to said axis. In another embodiment, said nodes are spread apart as said outer sheath expands, *i.e.,* the distance between said nodes increase. In another embodiment, the distance lying between said fibrils increases as said outer sheath expands. In another embodiment, the orientation of said nodes and/or fibrils changes as said outer sheath expands. In another embodiment, said sheath comprises ePTFE. In another embodiment, said coating comprises a hydrophilic component. In another embodiment, said therapeutic agent is a hydrophilic agent. In another embodiment, said therapeutic agent is a hydrophobic agent. In another embodiment, said therapeutic agent is paclitaxel. In another embodiment, said balloon further comprises a structural layer. In another embodiment, said structural layer comprises said coating and therapeutic agent. The microstructure of the sheath changes as said balloon expands.

The scope of the invention is defined by the claims. Any references in the description to methods of treatment refer to the compounds of the present invention for use in a method for treatment of the human (or animal) body by therapy. Other embodiments of the invention comprise a method of delivering a therapeutic agent to a desired location within a vessel comprising, inserting a catheter in a vessel, said catheter comprising an expandable member comprising a coating with a therapeutic agent, a sheath disposed around said expandable member, wherein said sheath has a variably permeable microstructure that prevents said coating from being transported through substantially all of said sheath in the unexpanded state, and wherein said coating and therapeutic agent are disposed between the surface of the expandable member and the sheath, advancing said catheter to a desired location within said vessel, and expanding the expandable member and sheath at the desired location within said vessel, and wherein substantially all of said sheath allows transfer of said coating and therapeutic agent from between the surface of the expandable member and the sheath to an area external to said sheath when said sheath is in an unexpanded state while preventing transfer of particles out of said sheath greater than about 25 microns in size. The expandable member is a medical balloon. In another embodiment, said sheath allows rapid transfer of said coating and therapeutic agent because said sheath rapidly wets out during expansion. In another embodiment, said sheath undergoes microscopic wetting in a vessel while said balloon and sheath are in the unexpanded state and being delivered to a desired location within a vessel. In another embodiment, said macroscopic wetting of the sheath is facilitated when said sheath is in contact with the vessel wall. In another embodiment, said sheath comprises a fluoropolymer. The sheath comprises a microstructure comprised of nodes interconnected by fibrils. In another embodiment, said nodes are aligned longitudinally to the longitudinal axis of said balloon catheter and said fibrils are aligned circumferentially to said axis. In another embodiment, said nodes are aligned circumferentially to the longitudinal axis of said balloon catheter and said fibrils are aligned longitudinally to said axis. In another embodiment, said nodes expand (elongate) said outer sheath expands. In another embodiment, said nodes are spread apart as said outer sheath expands. In another embodiment, said fibrils are spread apart as said outer sheath expands. In another embodiment, said sheath comprises ePTFE. In another embodiment, said therapeutic agent is a hydrophilic agent. In another embodiment, said therapeutic agent is a hydrophobic agent. In another embodiment, said therapeutic agent is paclitaxel. In another embodiment, said coating is hydrophilic. In another embodiment, said expandable member further comprises a structural layer. In another embodiment, said structural layer comprises said coating and therapeutic agent. In another embodiment, the microstructure of the sheath changes as said expandable member expands. In another embodiment, the hydrated or partially hydrated hydrophilic coating containing a therapeutic agent is tissue adherent, and thus, even after the expandable member is removed from the site, the drug continues to be absorbed into the tissue until the coating and drug dissipate from the site. This approach effectively increases the total drug delivery time to the tissue.

In another embodiment of the invention, said coating contains a hydrophobic drug that is complexed or sequestered by one or more solubilizing agents. In another embodiment, said solubilizing agent helps said hydrophobic drug transfer to a target tissue. In another embodiment, said solubilizing agent, when delivered to the intended tissue site, dissociates from said drug and the drug binds to tissue.

Another embodiment of the invention comprises a sheath disposed around a coating disposed about an expandable member where the sheath is purposefully under- or over-sized in diameter to further modulate fluid transfer through the outer sheath.

Another embodiment of the invention comprises a sheath disposed around a coating disposed about an expandable member wherein the sheath is purposefully modified with a wetting agent to facilitate wetting of said sheath in the unexpanded state. However, said modified sheath, even when wet-out, prevents drug transfer across said sheath in the unexpanded state.

In another embodiment, an expandable device such as a stent or stent-graft may be mounted to the "on-demand" agent elution construct of the invention, delivered to a site within the body where the expandable device is expanded and placed using the construct of the invention. The advantage of this application is that a therapeutic can be delivered to a treatment site along with another treatment device.

In another embodiment, following therapeutic treatment with the "on-demand" agent elution construct of the invention, an expandable device such as a stent, stent-graft, or other endoprosthesis may be placed in the treatment region, and the construct of the invention is used to "touch-up" or otherwise modify the degree to which at least a portion of the device is expanded.

In another embodiment, placement and/or "touching up" of an endoprosthesis with therapeutic agent elution constructs of the instant invention may comprise transferring a therapeutic agent from the construct to the endoprosthesis (*e.g.,* by absorptive transfer), whereby the endoprosthesis subsequently becomes a drug eluting endoprosthesis which operates therapeutically over short or long periods of time.

### BRIEF DESCRIPTION OF THE DRAWINGS

The exemplary embodiments of the present invention will be described in conjunction with the accompanying drawings. The accompanying drawings are included to provide a further understanding of the invention and are incorporated in and constitute a part of this specification, illustrate embodiments of the invention and together with the description serve to explain the principles of the invention. Figures are not drawn to scale.
Figure 1 depicts a general balloon catheter having an elongated tubular body with a balloon.
Figures 2A and 2B depict a cross-section of the drug delivery balloon of the invention in its first, unexpanded state (2A) and in its second, fully expanded, state (2B).
Figures 3A through 3D are scanning electron micrographs (SEMs) of two different outer sheaths comprising ePTFE. Figures 3A and 3B are SEMs of sheath 1, while Figures 3C and 3D are SEMS of sheath 2. Figures 3A and 3C respectively show sheath 1 and sheath 2 in their first state with a closed microstructure, and Figures 3B and 3D respectively show sheath 1 and sheath 2 in their second state with an open microstructure.
Figure 4 depicts a cross-section of the drug delivery balloon of the invention similar to Figure 2A with the addition of a structural layer.
Figure 5A depicts a catheter construct that can be used to deliver therapeutic agents. Figure 5B depicts a cross-section of the catheter construct of Figure 5A.
Figures 6A through 6D depict a method of using the catheter construct of Figure 5A.
Figures 7A and 7B depict degree of wetting of a device with a hydrophilic coating (Device 8a, Figure 7A) and a device without a coating (Device 8b, Figure 7B) after being submerged in blood in an unexpanded state.
Figures 8A and 8B depict degree of wetting of a device with a hydrophilic coating (Device 8a, Figure 8A) and a device without a coating (Device 8b, Figure 8B) after being submerged in blood and expanded within a rigid tube (serving as a mock vessel) to a pressure of 6 atmospheres for 1 minute and then deflated and rinsed.
Figures 9A and 9B depict degree of wetting of a device with a hydrophilic coating (Device 8a, Figure 9A) and a device without a coating (Device 8b, Figure 9B) after being submerged in blood and expanded in a rigid tube to a pressure of 12 atm.
Figures 10A and 10B depict Fourier Transform Infrared Spectroscopy (FTIR) interferograms of the PVA coating applied to Device 9 (Figure 10A) and released from Device 9 after expansion (Figure 10B).
Figures 11A through 11C depict degree of wetting of Device 9 when uninflated (Figure 11A), inflated to 12 atmospheres (atm) in blood without vessel contact (Figure 11B), and inflated to 12 atm in blood in a rigid tube serving as a mock vessel to provide vessel contact (Figure 11C).
Figure 12 depicts particulation from coated balloons with and without outer sheaths.
Figures 13A and 13B depict degree of wetting of Device 12 that was left unexpanded (Figure 13A) and expanded inside an artery (Figure 13B).
Figures 14A through 14D depict histological sections of arteries. Figure 14A depicts a light micrograph of a histological cross-section of the Control Artery of Example 12. Figure 14B shows a fluorescence micrograph of a histological cross-section of the Control Artery shown in Figure 14A. Figure 14C depicts a light micrograph of a histological cross section of the Test Artery of Example 12 after contact with a construct of the invention comprising Texas Red-labeled dextran. Figure 14D shows a fluorescence micrograph of a histological cross-section of the Test Artery shown in Figure 14C.
Figures 15A and 15B show degree of wetting of Device 13 after *in vivo* incubation in canine arteries in unexpanded (Figure 15A) and expanded (Figure 15B) states.

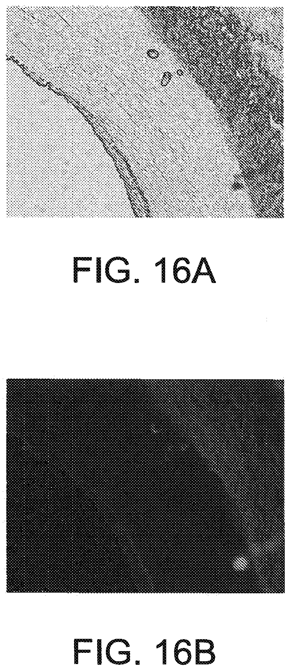

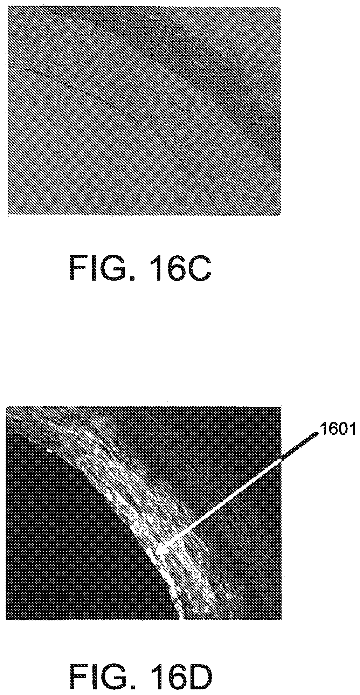

Figures 16A through 16D depict histological sections of arteries. Figure 16A depicts a light micrograph of a histological cross-section of the Control Iliac Artery of Example 13. Figure 16B shows a fluorescence micrograph of a histological cross-section of the Control Iliac Artery shown in Figure 16A. Figure 16C depicts a light micrograph of a histological cross section of the Test Iliac Artery of Example 13 after contact with a construct of the invention comprising Texas Red-labeled dextran. Figure 16D shows a fluorescence micrograph of a histological cross-section of the Test Iliac Artery shown in Figure 16C.
Figure 17 shows Device 14 of Example 14 after expansion to 6 atm (Figure 17A) and 12 atm (Figure 17B) in blood in a rigid tube without prehydration in blood.

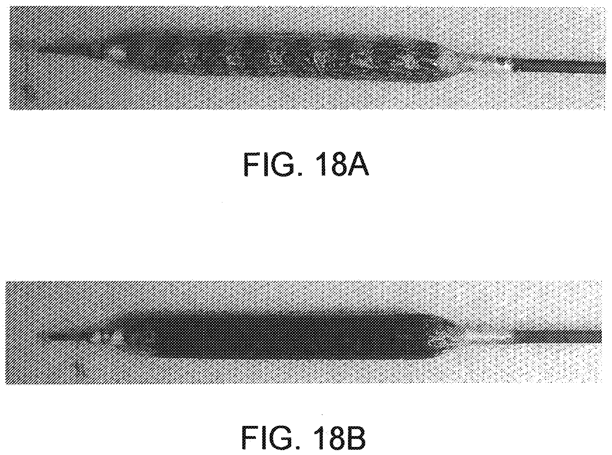

Figure 18 depicts degree of wetting of Device 8a after prehydration in blood at first state and then expansion in blood in a rigid tube at 6 atm for 1 minute (Figure 18A), and finally expansion in blood in a rigid tube at 12 atm for 1 minute (Figure 18B).
Figure 19 depicts degree of wetting of Device 15 of Example 15 after expansion in a canine femoral artery *in vivo.*
Figure 20A, 20B, and 20C show the degree of wetting of Device 16 when uninflated (Figure 20A), inflated to 6 atm in a rigid tube in blood (Figure 20B), and inflated to 12 atm in a rigid tube in blood (Figure 20C).
Figure 21 depicts treatment averages of drug concentration (nanogram (ng) drug per gram (g) tissue, n=3 arteries per treatment) in tissue segments proximal to, within the treatment site, distal to, or remote from tissue treated by constructs of the invention as described in Example 18.
Figure 22 depicts 24 hours treatment averages of paclitaxel concentration (ng drug per g tissue, n=2 arteries per treatment) in tissue segments proximal to, within the treatment site, distal to, or remote from tissue treated by constructs of the invention as described in Example 21.
Figure 23 depicts 1 hour treatment averages of paclitaxel concentration (ng drug per g tissue, n=3 arteries per treatment) in tissue segments proximal to, within the treatment site, distal to, or remote from tissue treated by constructs of the invention as described in Example 21.

### DETAILED DESCRIPTION OF THE EMBODIMENTS

The invention is directed to a balloon catheter comprising an agent eluting construct for delivery of at least one therapeutic agent to a desired site within a mammalian body. The therapeutic agent elution construct of the instant invention comprises additional structures which ensure drug delivery to the target site without significant drug loss during device tracking to the target site and without particulation of the agent. Said agent elution construct comprises an expandable member. In another embodiment, said expandable member is a medical balloon. (As used herein balloon and medical balloon are used interchangeably, unless otherwise noted).

For clarity, the figures, the description and the examples describe and depict an agent elution construct comprising a medical balloon.

Reference will now be made in detail to embodiments of the present invention, examples of which are illustrated in the accompanying drawings.

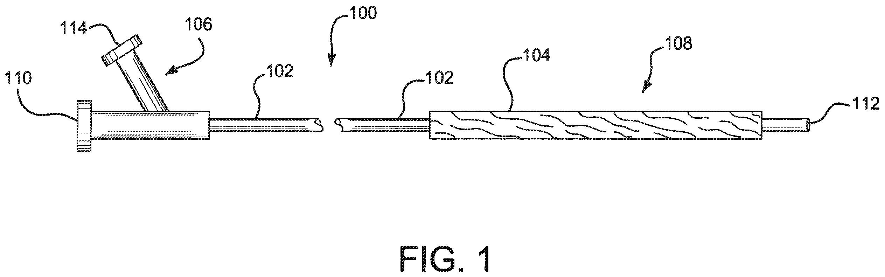

Figure 1 is illustrative of a balloon catheter 100 having an elongated tubular body 102 with a balloon 104. In one embodiment balloon 104 may be a length adjustable balloon.

The elongated tubular body 102 has a proximal control end 106 and a distal functional end 108. The balloon catheter also has a proximal guidewire lumen 110 that extends through the length of the elongated tubular body 102 and exits the distal end at a guidewire port 112. The balloon catheter shown is an "Over The Wire" configuration, as commonly known in the art. Alternatively, the catheter could have a guidewire port located midway between proximal and distal ends and therefore have a "Rapid Exchange" configuration, as commonly known in the art. The balloon catheter 100 also incorporates a proximal inflation port 114 that allows fluid communication between the inflation port 114 and the lumen of the balloon 104. The length and inner and outer diameter of the tubular body are selected based upon the desired application of the medical device. The tubular body generally has a circular cross-sectional configuration. However, oval and other cross-sectional configurations can also be used. In one embodiment, said balloon catheter is compatible with 0.038", 0.035", 0.018" or 0.014", 0.010", or similar conventional guidewires.

The tubular body must have sufficient structural integrity to permit the medical device to be advanced to distal vascular locations without bending or buckling upon insertion. Various techniques are known for manufacturing the tubular bodies. In one embodiment, the tubular body is manufactured by extrusion of a biocompatible polymer.

The invention is also directed to an expandable balloon catheter that delivers a therapeutic agent to a vascular site using consistent "on-demand" delivery while not substantially eluting or releasing therapeutic agent(s) while the device is being tracked to a desired location within the vasculature. The balloon catheter of the current invention comprises an expandable member with (or without) a structural or substrate layer over the expandable member, at least one hydrophilic coating comprising at least one therapeutic agent disposed on the expandable member or structural layer, and an outer sheath comprising a variably permeable microstructure. During use, the underlying hydrophilic coating becomes hydrated or partially hydrated and facilitates fluid transfer across the outer sheath. However, said outer sheath's closed microstructure in the unexpanded state prevents unwanted, premature release of said therapeutic agent in the unexpanded state. Upon expansion, the orientation or configuration of the microstructure of the material comprising the outer sheath, which is disposed over the expandable member, transforms from a substantially closed microstructure to a substantially open microstructure allowing the hydrated or partially hydrated coating to be transferred outward. This feature of the microstructure of the material is a material having a variably permeable microstructure. Once the hydrated or partially hydrated hydrophilic coating passes through the outer sheath, the therapeutic agent is delivered to the treatment site. In one embodiment, the hydrated or partially hydrated coating comprises a therapeutic agent and once the outer sheath is expanded, the therapeutic agent transfers through the sheath. Said expandable member is a medical balloon. Said outer sheath has a relatively closed microstructure when there is no strain on the outer sheath. Said sheath has a more open microstructure when said sheath is strained (*i.e.,* diametrically strained). The strain on said outer sheath can be exerted by said expandable member during expansion.

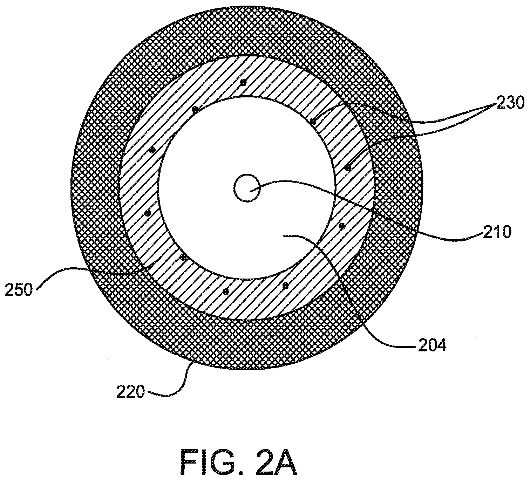

The agent elution construct of the invention comprises several aspects to help control delivery of therapeutic agents from an expandable member. Figure 2A is a cross-section of an agent elution construct comprising a balloon in its first, uninflated, state. The construct comprises a balloon 204, a hydrophilic coating 250 on balloon 204 and an outer sheath 220. Hydrophilic coating 250 further comprises at least one therapeutic agent 230. Also depicted is guidewire lumen 210 that extends through the length of the balloon. In one embodiment, said hydrophilic coating is substantially dehydrated prior to device insertion into the vasculature. In another embodiment, the outer sheath 220 is made from a material having a variably permeable microstructure. In another embodiment, outer sheath 220 is wrapped or folded over hydrophilic coating 250 at a first, uninflated diameter.

Materials which may exhibit variably permeable microstructures are known to the art. These include, but are not limited to, fibrillated structures, such as expanded fluoropolymers (for example, expanded polytetrafluoroethylene (ePTFE)) or expanded polyethylene (as described in U.S. Patent 6,743,388); fibrous structures (such as woven or braided fabrics; non-woven mats of fibers, microfibers, or nanofibers; materials made from processes such as electrospinning or flash spinning; polymer materials consisting of melt or solution processable materials such as fluoropolymers, polyamides, polyurethanes, polyolefins, polyesters, polyglycolic acid (PGA), polylactic acid (PLA), and trimethylene carbonate (TMC), and the like; films with openings created during processing (such as laser- or mechanically-drilled holes); open cell foams; microporous membranes made from materials such as fluoropolymers, polyamides, polyurethanes, polyolefins, polyesters, PGA, PLA, TMC, and the like; porous polyglycolide-co-trimethylene carbonate (PGA:TMC) materials (as described in U.S. Patent 8,048,503 and incorporated herein by reference); or combinations of the above. Processing of the above materials may be used to modulate, enhance or control permeability between a first, closed state and second, expanded. Such processing may help close the microstructure (thus lower permeability) in a first state, help open the microstructure in a second state, or a combination of both. Such processing which may help close the microstructure may include, but is not limited to: calendaring, coating (discontinuously or continuously), compaction, densification, coalescing, thermal cycling, or retraction and the like. Such processing that may help open the microstructure may include, but is not limited to: expansion, perforation, slitting, patterned densification and/or coating, and the like. In another embodiment, said materials comprise micropores between nodes interconnected by fibrils, such as in ePTFE. In another embodiment, said material comprises micropores in an essentially nodeless ePTFE, as described in U.S. Patent 5,476,589.

In another embodiment of the invention, the surface(s) or outward configuration of the sheath material may be modified with textures, protrusions, spikes, scorers, depressions, grooves, coatings, particles, and the like. These may serve various purposes such as to modify tissues into which therapeutic agents will be (or have been) delivered, control placement of the system of the invention, and direct fluid transfer. Such textures may help in increased transfer of a therapeutic agent onto, more deeply and/or into deeper tissues. In addition, coatings may aid in microscopic wetting of said sheath material. In one embodiment, said coating of said sheath material comprises crosslinked polyvinyl alcohol (*see, e.g.,* U.S. Patent 7,871,659). In another embodiment, said coating of said variably permeable microstructure material comprises a heparin coating, such those described in U.S. Patents 4,810,784 and 6,559,131.

In another embodiment of the invention, the location(s) of the permeable microstructure may be varied. For example, a sheath may be constructed such that only a portion of its microstructure is variably permeable. Such a configuration may be desirable where fluid transfer is not desired to occur, for example, at one or both of the ends of the expandable medical device of the invention. This may be desirable where multiple drug eluting devices will be used in a specific anatomy, and it would be undesirable to overlap treatments sites, *i.e.,* delivering too much drug to a particular site.

In another embodiment, the sheath may contain or be marked with radiopaque markers or be constructed to be radiopaque in its entirety. Such radiopaque indicators are used by clinicians to properly track and place an expandable medical device of the invention.

As used herein, the term "variably permeable microstructure" refers to a structure or material with a resistance to fluid transfer at a first state that is higher than the resistance of the same structure or material at a second state with such resistance varying between the two states. One skilled in the art will appreciate various methods which characterize the change in permeability from testing at a first state and comparing to testing done at a second state. These methods include, but are not limited to, characterizations of air or liquid flux across the microstructure at a given pressure differential, characterization which determines the pressure differential at which different fluids strike through the microstructure such as Water Entry Pressure or Bubble Point, characterization of porosity, and visual characterization such as inter-nodal or inter-fibril spacing as measured from an image (*e.g.* from a scanning electron microscope or light microscope). One nonlimiting embodiment of a variable permeable material comprises a material that has a substantially closed microstructure when the material is not under a strain and has a more open microstructure when the material is strained.

As used herein, the terms "micropores" and "microporous" refer to openings in materials, for example the area between ePTFE nodes and fibrils. Usually, as in the case of ePTFE, these micropores contain air when the material is not "wetted".

As used herein, the terms "wet", "wet-out" and "wetted" refer to the displacement of air in a microporous material by a fluid. Wetting of a material lowers the resistance to subsequent fluid transfer and facilitates the flow of fluids though the microporous material. Furthermore, these microporous materials are intended to be open cell structures, meaning the micropores are interconnected, and not closed cell structures. This allows fluid to flow through the material. Capillary effects may also play an important role in fluid flow though the material as wetting occurs, especially for highly porous materials with small interconnected pores. Wetting can be accomplished with the aid of one or more surfactants added to the fluid. The surfactant can absorb onto the fluid-vapor, solid-fluid, and solid-vapor interfaces, which in turn modifies the wetting behavior of hydrophobic materials. The wetting will also depend on the viscosity to the fluid.

As used herein, the term "coating" refers to one or more materials disposed on the surface of a substrate. In the present invention the substrate may include the structural layer or substrate or expandable member or outer sheath. Said coating may lie completely on the surface or may be incorporated, in whole or in part, within the openings or pores present in a substrate. The latter coating configuration is commonly referred to in the art as "imbibed" or "filled" materials.

As used herein, the term "dry coating" or "dehydrated coating" refers to the inability of the coating alone to sufficiently wet the outer sheath by the displacement of air in a microporous material. Some dry coating embodiments may be formulated with at least one component that is in a liquid state in its pure form capable of causing wet-out, but when combined with additional components results in a dry coating.

As used herein, the term "vessel" refers to any luminal or tubular structure within the body to which these constructs can be utilized. This includes, but not limited to, vascular blood vessels, vascular defects such as arteriovenous malformations, aneurysm, or others, vessels of the lymphatic system, esophagus, intestinal anatomy, sinuous cavity, uterus, or other. The embodiments of the present invention are also suitable for the treatment of a malignant disease (*i.e.* cancer) within or associated with a vessel

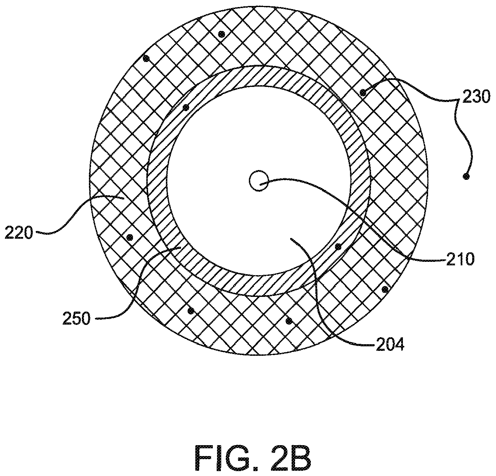

Figure 2B depicts the same construct as Figure 2A, except that the agent elution construct is at its second, expanded, state. This Figure depicts an inflated balloon 204, a hydrophilic coating 250 on the balloon 204 and an outer sheath 220, depicting a more open microstructure (*e.g.,* if said sheath comprises ePTFE, said open microstructure comprises increased distance between the nodes and/or increased distance between the fibrils and/or changes in orientation of the fibrils and/or nodes (fibril and/or node re-orientation)). The hydrophilic coating 250 further comprises at least one type of therapeutic agent 230. Also depicted is guidewire lumen 210 that extends through the length of the balloon. As seen in this Figure, therapeutic agent 230 is passing from the surface of balloon 204, into and through the outer sheath 220, and out of the balloon construct. It will be understood that the hydrophilic coating 250 may, in some embodiments, pass into and through the outer sheath 220, and out of the balloon construct. In another embodiment, upon expansion, the hydrophilic coating 250 passes into and through the outer sheath 220 in a hydrated or partially hydrated state. In another embodiment, outer sheath 220 is wetted after expansion. In another embodiment, said sheath is fully wetted before expansion. In another embodiment, said sheath is partially wetted before expansion. In another embodiment, coating 250, once external to the sheath 220, is tissue adherent and remains adhered to the target tissue even after the device is removed. This embodiment allows for continued drug transfer from the adherent coating at the tissue interface until the tissue adherent coating dissipates from the target tissue, as described in the co-pending and co-assigned U.S. Patent Publication 20100233266. In another embodiment, the coating comprises a thixotropic gel.

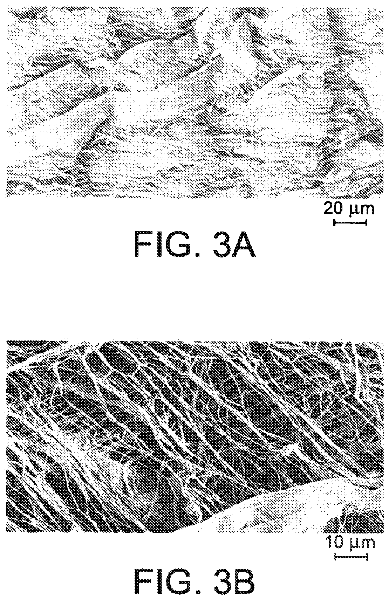

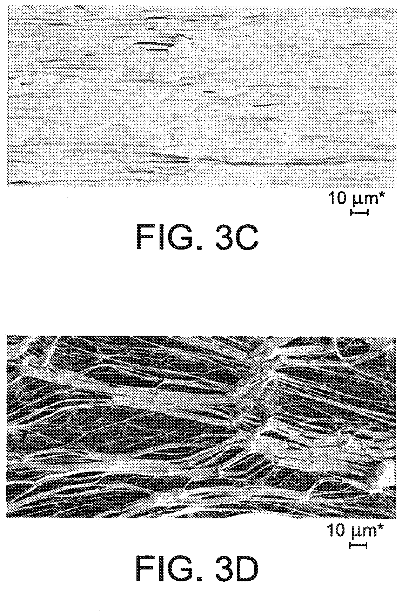

Figures 3A, 3B, 3C, and 3D are scanning electron micrographs (SEMs) of two different outer sheaths with variably permeable microstructures that comprises ePTFE. Specifically, Figures 3A and 3C respectively show outer sheath 1 and outer sheath 2 when these agent elution constructs are in their first, unexpanded, state. As seen in 3A and 3C, the microstructures of these outer sheaths are relatively compact with fibrils and nodes positioned close to one another. There are very few and/or very small micropores in these structures.

Figures 3B and 3D show outer sheath 1 and outer sheath 2 of Figures 3A and 3C, respectively, in their second, expanded, state. As shown in these micrographs, the microstructures are now considerably more open than that seen in Figures 3A and 3C. In other words, the distance between nodes and/or the distance between fibrils have increased. As can be seen in these Figures, distance between nodes has increased and the orientation of the fibrils has changed. As a result, micropores are larger (as compared to Figures 3A and 3C). Since the micropores of Figures 3B and 3D are larger than the micropores of Figures 3A and 3B, fluid can penetrate and (at least partially) displace the air within the micropores. When this occurs, the outer sheath is wetted.

Most microporous materials will eventually wet-out with body fluids following implantation. However, this process may require significant time (hours to days). In the case of some fluoropolymers, such as ePTFE, its hydrophobic nature can greatly slow the process of replacing air with fluid, which may slow or completely restrict therapeutic agent release from a coated expandable member, *e.g.* balloon, underlying under the outer sheath. However, if the ePTFE is wet too quickly, which can occur when the micropores are too large, then premature drug release may occur before balloon catheter is positioned at the desired location.

In one embodiment, one of the disclosed inventions addresses this dilemma by the use of a "switch" mechanism that controls drug elution as a function of expansion of the expandable member. This controlling switch mechanism results from the novel combination of an expandable microporous material in the outer sheath with a dehydrated hydrophilic coating underneath the outer sheath. In one embodiment, once the hydrophilic coating begins to become, or is fully hydrated, the tight porosity of the outer sheath at its first state, as shown in Figures 3A and 3C, will serve as a bulk fluid transfer barrier to the hydrated or partially hydrated coating and/or the therapeutic agent associated therewith. However, upon expansion (*i.e.,* inflation of the medical balloon), the combination of the opening of the micropores, as shown in Figures 3B and 3D, with pressure-driven expansion and the hydrated or partially hydrated hydrophilic coating rapidly displacing air within at least a portion of the outer sheath (*i.e.,* the coating wets-out the outer sheath), transfer of the coating or coating and therapeutic agent occurs. Such transfer occurs without particulation. At the same time, as the outer sheath expands, body fluids will also displace air within the outer sheath allowing for an influx of body fluids which will further hydrate the coating and which, in turn, help the coating displace the air in the outer sheath. In this embodiment, the hydrophilic coating is selected from a group that while being hydrophilic is also compatible with the sheath material to affect sheath wetting and subsequently provide for efficient coating transfer into and through the microstructure of the sheath. Such compatibility of coating to sheath material(s) can be tailored to meet the desired wetting characteristics (*see, e.g.,* U.S. Patent 5,874,165).

This "switching" phenomenon is possible due to a unique combination of a dehydrated hydrophilic coating which contains a therapeutic agent combined with a variably permeable and expandable outer sheath. The combination results in an agent eluting construct that prevents the transfer of therapeutic agent at first state but which allows for transfer of therapeutic agent at its second state where there the agent eluting construct exhibits an increase in pore size of the outer sheath. Without being bound to a particular theory, therapeutic agent transfer may be related to two main drivers: the hydrophilic coating acting as a wetting agent; and shear forces at the interfaces of the outer sheath and coating as expansion occurs.

The switch mechanism represents a dynamic continuum as the variably permeable microstructure of the outer sheath changes in response to wetting and/or an expansion force. When the microstructure opens in response to said expansion force, there is also sufficient force to drive fluid transfer. When this occurs the agent elution construct of the invention is said to be "switched" from an "off" state in which the therapeutic agent and/or coating cannot pass through the sheath to an "on" state in which it can. It will be understood that the agent elution construct of the invention is not binary in its operation. Instead, while fluid transfer may be initiated at a discrete point in time, transfer rates will vary in accordance with the degree (and period of time) at which the microstructure of the outer sheath changes, *e.g.,* opens and/or closes, is wet, or remains partially wetted, etc. Such changes may be controlled, for example, by varying the pressure of a semi-compliant expandable member.

In the embodiment in which the outer sheath comprises ePTFE, when the balloon is in its first state, the ePTFE comprising outer sheath has a substantially closed microstructure, as shown in Figures 3A and 3C, because said sheath is collapsed around said balloon. Thus, the micropores are very small and will not readily: allow body fluids to substantially traverse the outer sheath, allow fluid transfer of the underlying coating (even if hydrated or partially hydrated), or allow for particulation of the therapeutic agent and/or coating during the time course of typical clinical usage of the therapeutic intervention. (As will be described below, there may be partial and/or full pre-hydration of the underlying coating due to a small amount fluid transfer inward through the sheath or due to the addition of wetting agent(s) to the outer sheath). Once the drug delivery balloon of the invention is at the desired location in the patient's body, the balloon is inflated, thus expanding the outer sheath to an open microstructure, as shown in Figures 3B and 3D. As the microstructure expands, micropores become larger, bodily fluids (*e.g.,* blood, serous fluid) displace air in the microstructure, and these fluids begin to flow inward through the outer sheath. The underlying hydrophilic coating is now exposed to an influx of said body fluids. As the body fluids hydrate the hydrophilic coating, the coating, in turn, will facilitate rapid wetting of the outer sheath by body fluids. Without being bound to a particular theory, this mechanism provides a feed-back loop that imparts rapid wet-out of the outer sheath and hydration of the hydrophilic coating. As the outer sheath wets out and the hydrophilic coating hydrates, the therapeutic agent is transported through the outer sheath by bulk fluid flow of the hydrated or partially hydrated coating as the balloon is inflated. This, in turn, will cause further wetting of the ePTFE and further reduce the barrier to transfer of the therapeutic agent. This embodiment enables consistent, controlled on-demand drug delivery to a target site (*e.g.* a body vessel). In another embodiment, the hydrated or partially hydrated coating will be forced through the outer sheath by the pressure applied by the expanding balloon.

In another embodiment, a cover may surround all or a portion of the drug eluting balloon catheter of the present invention. Such covers may work to isolate the balloon catheter surface from the external environment during shipment and storage or during use, *e.g.,* during tracking of the catheter to a treatment site. In one embodiment, the cover comprises a film cover held in place by stitching, for example the stitching as disclosed in U.S. Patent 6,352,553. In another embodiment, the cover comprises a film which can be everted off of the drug eluting balloon.

Although not part of this invention, an expandable device, such as a stent or stent-graft, may be mounted to the agent elution construct of the invention, delivered to a site within the body where the expandable device is expanded and placed. The advantage of this application is that a therapeutic agent can be delivered to the treatment site at the same time as said expandable device is being delivered. This prevents clinicians from having to switch between a stent delivery balloon and a drug delivery balloon. In one embodiment, said stent is made from a balloon expandable material, such as stainless steel. In another embodiment, said stent is made from a self-expanding material, such as Nitinol. In another embodiment, said stent is made from a biodegradable material, such as a biodegradable polymer, metal or metal alloy. In another embodiment, said stent comprises a graft. In another embodiment, said graft comprises ePTFE.

In another embodiment, a hydrophilic coating or a hydrophilic coating in combination with a therapeutic agent is applied to only a portion of an expandable member, *e.g.,* the surface of the balloon, in a discontinuous fashion. Upon "switching" the coating and/or therapeutic agent are delivered to a discrete or more localized site external to the outer sheath. In contrast, when the coating and/or therapeutic agent is applied in an even distribution to the entire surface of the expandable member, expansion (*e.g.* "switching") enables uniform delivery of the coating and/or therapeutic agent from the entire circumference of the expandable member.

As described in the examples below, fluid transfer through the outer sheath is also assisted by touching the expanding outer sheath against the vessel wall. In this situation, outer sheath's contact with the vessel may cause the surrounding body fluid pressure to exceed the fluid entry pressure of the outer sheath. In other words, the vessel may push fluid external to the outer sheath into the micropores of the sheath. Thus, in one embodiment, fluid transfer of the outer sheath is facilitated when said sheath is in contact with the vessel wall.

As also described in examples below, the outer sheath can be prepared with a second diameter that provides a resistance to growth above nominal diameter of the underlying expandable member, *e.g.,* balloon. This may, in turn, help to facilitate rapid wetting of the outer sheath which aids in fluid/coating/therapeutic agent transfer through the outer sheath. Thus, in one embodiment, as the balloon is inflated to nominal diameter, the hydrated or partially hydrated coating is trapped between an underlying balloon which is growing and an outer sheath that is resisting such growth. This provides some of the driving force for bulk fluid transfer of the hydrated or partially hydrated coating through the outer sheath.

In addition, due to the dimensions of the microstructure of the outer sheath as the balloon is tracked to the treatment site and during inflation, substantially no coating particles greater than about 25 µm are released. In another embodiment, a very small amount of coating particles greater than about 5 µm, about 10 µm, about 15 µm, or about 25 µm are released through the outer sheath. Thus, particulation of the drug and/or the coating matrix is minimized. In another embodiment, said outer sheath expands, but does not tear or break.

Thus, one embodiment of the invention comprises the drug delivery system comprising an expandable member (a balloon), which may comprise a structural layer and/or a substrate, at least one dehydrated or partially dehydrated hydrophilic coating containing at least one therapeutic agent, said coating located on the expandable member or structural layer and/or substrate, and an outer sheath with a variably permeable microstructure which is expandable by the expandable member. In its unexpanded state, the sheath is of a lower permeability. As it is expanded, it becomes more permeable. In one embodiment, the hydrophilic coating becomes at least partially hydrated prior to the sheath being expanded, but the coating and the therapeutic agent do not pass (or substantially pass) through the outer unexpanded sheath. In another embodiment, a driving force sufficient to transfer the coating across the sheath is necessary. In another embodiment, as the sheath is expanded and its microstructure opens, the hydrated or partially hydrated coating lowers the fluid entry pressure of the sheath and this, in combination with increasing pore size of the sheath and a higher driving force supplied by the expandable member, causes fluid transfer of the coating and/or the therapeutic agent through the sheath. Once the hydrated or partially hydrated hydrophilic coating passes through the sheath, the therapeutic agent in the coating is delivered to the treatment site. In another embodiment of the invention, the lowering of the fluid entry pressure of the sheath is effected *via* wetting of the outer sheath by a wetting agent applied to said outer sheath. In another embodiment, the wetting agent on said outer sheath comprises poly(vinyl alcohol) (PVA) or a heparin coating.

In another embodiment of the invention, a hydrophobic drug is sequestered by or complexed with one or more solubilizing agents such that when delivered to the intended tissue site the drug dissociates from the solubilizing agent and binds to tissue. Such solubilizing agents are known in the art (*see, e.g.,* U.S. Patent Publication 20080118544).

Another embodiment of the invention comprises a balloon catheter comprising, an expandable member, a coating comprising a therapeutic agent disposed around said expandable member, a sheath disposed around said coating, wherein said sheath has a variably permeable microstructure that initially prevents or limits unintended transfer of therapeutic agent through said sheath, wherein said coating and therapeutic agent are disposed between the surface of the expandable member and the sheath, and wherein when said expandable member and sheath are expanded, said sheath allows transfer of said coating and therapeutic agent to an area external to said sheath while preventing transfer of particles out of said sheath greater than about 25 microns in size. In another embodiment, said sheath rapidly wets out during expansion, and said sheath allows rapid transfer of said coating and therapeutic agent. In another embodiment, said sheath undergoes microscopic wetting in a vessel while said balloon and sheath are in the unexpanded state and being delivered to a desired location within a vessel. In another embodiment, bodily fluids substantially wet-out the sheath when said sheath is being expanded. In another embodiment, said hydrophilic component also wets the sheath when said sheath is being expanded. In another embodiment, substantially all of said sheath is wet by the time said sheath is fully expanded. In another embodiment, fluid external to said sheath is allowed to flow through said sheath, and contact said therapeutic agent. In another embodiment, said wetting of said sheath is facilitated when said sheath is in contact to the vessel wall. In another embodiment of the invention, wetting of the outer sheath is facilitated by a wetting agent applied to said outer sheath. In another embodiment, the wetting agent of said sheath comprises poly(vinyl alcohol) (PVA) or a heparin coating. In another embodiment, said sheath comprises a fluoropolymer. The sheath comprises a microstructure comprised of nodes interconnected by fibrils. In another embodiment, said nodes are aligned longitudinally to the longitudinal axis of said balloon catheter and said fibrils are aligned circumferentially to said axis. In another embodiment, said nodes are aligned circumferentially to the longitudinal axis of said balloon catheter and said fibrils are aligned longitudinally to said axis. In another embodiment, said nodes are spread apart as said outer sheath expands. In another embodiment, said fibrils are spread apart as said outer sheath expands. In another embodiment, said coating comprises a hydrophilic component. In another embodiement said coating comprises at least one compound selected from the group consisting of benzethonium chloride, poloxamer-188, polyethylene glycol, sodium salicylate, and hydroxypropyl-β-cyclodextrin. In another embodiment, said therapeutic agent is a hydrophilic agent. In another embodiment, said therapeutic agent is a hydrophobic agent. In another embodiment, said therapeutic agent is paclitaxel or a taxane domain-binding drug. In another embodiment, said expandable member further comprises a structural layer. In another embodiment, said structural layer comprises said coating and therapeutic agent. In another embodiment, the microstructure of the sheath changes as said expandable member expands.

In some embodiments, if the sheath and/or the structural layer are composed of a thin film wherein said film comprises a microstructure of nodes interconnected by fibrils, then unlike extruded tubes, said nodes will not pass through the entire thickness of said structural layer and/or sheath. Said nodes are only as thick as the film. Accordingly, the along the thickness of a film tube (*i.e.,* a tube made of wrapping a film) in which there are several passes of a film, there will be a number nodes only as thick as the film and placed randomly along the thickness of said film tube. For the purposes of this invention, the term "nodes aligned circumferentially" means that if a majority of nodes have a length that is longer than the width of said node, then the length of said node will be aligned in the circumferential direction of a wrapped tubular construct, such as a structural layer and/or sheath (*see, e.g.* Figure 3C). For the purposes of this invention, the term "nodes aligned longitudinally" means that if a majority of nodes have a length that is longer than the width of said node, then the length of said node will be aligned to the longitudinal axis of a wrapped tubular construct, such as a structural layer and/or sheath. In another embodiment, if a tubular construct made from a film wherein said film comprises a microstructure of nodes interconnected by fibrils and said nodes are aligned in a circumferential direction, then upon radial expansion of said tube, said nodes increases in length. Methods of making tubes made from films are described below.

Another embodiment of the invention comprises a sheath disposed around a coating disposed on an expandable member where the sheath is purposefully under- or over-sized in diameter to further modulate fluid transfer through the outer sheath. By "under-sized" it is meant a sheath which will not expand greater than the nominal diameter of the underlying expandable member without stretching. This is useful because it can prevent the balloon from bursting and also constrain the volume of coating and/or therapeutic agent, helping to drive transfer of the coating and/or therapeutic agent through the outer sheath. By "over-sized" it is meant a sheath expandable beyond (or constructed to be) of a diameter larger than the nominal diameter of the underlying expandable member.

In another embodiment, the variably permeable microstructure of the outer sheath can be selected or controlled to modify how inflation pressure affects the release of the therapeutic agent. For example a sheath may be selected which allows transfer of the coating and/or therapeutic agent over a narrow range of inflation pressures. Conversely, the sheath may be constructed to provide transfer over a larger range of inflation pressures. In addition, the sheath may be constructed to tailor transport in conjunction with changes in diameter of the agent eluting device due to changes in inflation pressure. The desired variability can, for example, be achieved by using different materials for the outer sheath and/or different thickness of said materials and/or different orientations of said materials and/or different processing of said materials.

As used herein, the terms "rapid" and "rapidly" refer to a clinically relevant timeframe, *e.g.,* less than about 5.0 minutes. In another embodiment, the terms "rapid" and "rapidly" are defined herein to mean about 90, about 60, about 50, about 45, about 30, about 20, or about 10 seconds.

In some embodiments, the outer sheath will not be fully wet out. As further described below, very small, microscopic areas of the outer sheath can be wetted out. As used herein the term, "microscopic-wetting" refers to small areas of the outer sheath which wet, (*i.e.,* air is replaced by liquid fluids) but these wet areas are so small that such wetting, that may be indicated by translucence of the wetted material (depending on the material), will not be visible to naked eye. In one embodiment, the outer sheath is composed of ePTFE which may undergo microscopic wetting, and thus, the outer sheath will not become translucent. Microscopic-wetting can occur when the outer sheath is in its first diameter and may contribute to pre-hydration of the coating. As will be further described below, this occurs in areas of the outer sheath where the micropores are large enough to allow air displacement by fluids.

As used therein the term "macroscopic wetting" is when the outer sheath is wet and wetting can be detected by the naked eye, for example, by at least a portion of an ePTFE comprising outer sheath becoming translucent.

In some instances, the outer sheath, by design or due to variations in manufacturing, may have pores that allow microscopic wetting by fluids. This allows the fluids to enter through the outer sheath and to the coating, thus pre-hydrating the coating. Therefore, as the agent elution construct of the invention is being tracked to the desired location, body fluids may be pre-hydrating the dehydrated or partially-dehydrated hydrophilic coating. The examples below suggest that it may be helpful to pre-soak the balloon construct of the invention in order to achieve rapid and complete wet-out of the outer sheath. Thus, one embodiment of the invention provides for pre-hydration of the hydrophilic coating provided by body fluids as the agent elution construct of the invention is being tracked to the target site. As used herein the term "pre-hydration" means that the hydrophilic coating is hydrated or partially hydrated while the expandable member and the outer sheath are in their first, unexpanded, state. In this embodiment, in their first, unexpanded, state, the coating and/or therapeutic agent will not be released to an area external to the outer sheath in significant quantities. It will be appreciated by one of skill in the art that pre-hydration might be accomplished in whole or in part during preparation of the device prior to introduction into a patient.

As discussed, it may be beneficial to have some fluid transfer into and through the outer sheath in order to have pre-hydration of the hydrophilic coating, depending, *inter alia,* on the coating and/or therapeutic agent formulation. However, relying on pores due to variability in manufacturing of a microporous structure, such as ePTFE, may not be sufficient to induce pre-hydration of the hydrophilic coating and rapid wet-out of the outer sheath during expansion. Thus, in one embodiment, a portion of the outer sheath (exterior area) is treated with a wetting agent. Suitable wetting agents include a hydrophilic coating or others well known in the art. That portion of the sheath "imbibed," "filled" or treated by the wetting agent will instantaneously (*i.e.,* in less than about 10 seconds) wet-out when contacted by bodily fluids ("point wetting"). In turn, this allows said bodily fluids to pass through the sheath and into the hydrophilic coating, thus causing said coating to hydrate or partially hydrate. In another embodiment, the hydrophilic coating will fully hydrate, even if such "point wetting" is employed. This is because even small amounts of bodily fluids in contact with the coating are rapidly transported throughout the coating, hydrating the coating to some degree. Because the rest of the sheath remains unexpanded and/or unwetted, the now hydrated or partially hydrated coating remains substantially on the inside of the outer sheath until it is expanded by mechanisms described above. In another embodiment, said fluid is a vapor that can pass through the outer sheath and condense on the dehydrated coating. In this embodiment, the outer sheath may not become wet but allows for coating hydration. In another embodiment, conditioning the outer sheath with a wetting agent can be varied and/or patterned along the length and surface area of the outer sheath so that wetting of said outer sheath is uneven. This may help in adjusting the rate of wetting, the rate of delivery and/or amount of said therapeutic agent/coating delivered. In one embodiment, the outer sheath is partially conditioned with a wetting agent in a pattern along the outer sheath's surface to allow for "near instantaneous" wetting (*i.e.,* in less than about 20 seconds).

In other embodiments, the entire outer sheath is treated, imbibed and/or filled with a wetting agent that can be cross-linked to allow instantaneous wetting (*i.e.,* in less than about 10 seconds) of the outer sheath following contact with an aqueous medium, as described in U.S. Patent 7,871,659, and U.S. Patent 5,897,955. In one embodiment, said wetting agent includes, but not limited to poly(vinyl alcohol) polyethylene glycol, heparin, heparin coatings (such as those described in U.S. Patent 6,461,665), polypropylene glycol, dextran, agarose, alginate, polyacrylamide, polyglycidol, poly(vinyl alcohol-co-ethylene), poly(ethyleneglycol-co-propyleneglycol), poly(vinyl acetate-co-vinyl alcohol), poly(tetrafluoroethylene-co-vinyl alcohol), poly(acrylonitrile-co-acrylamide), poly(acrylonitrile-co-acrylic acid-co-acrylamidine), polyacrylic acid, poly-lysine, polyethyleneimine, polyvinyl pyrrolidone, polyhydroxyethylmethacrylate, and polysulfone, and their copolymers, either alone or in combination. However, the hydrated or partially hydrated coating and/or therapeutic agent will not be substantially transferred (or only a small amount may transfer) through the outer sheath in its first, unexpanded state because the outer sheath has closed microstructure and/or because there is no back pressure forcing the hydrated or partially hydrated coating to be transferred (*e.g.* pushed) outward.

In another embodiment, said outer sheath has small perforations, holes, slits, larger pores, or any other imperfection that allows body fluids to pre-hydrate the hydrophilic coating, without substantially allowing any therapeutic agent or coating particles to be released into the bloodstream while the balloon is in the first state. In another embodiment, controlled release of the inflation media from the underlying balloon may also serve to pre-hydrate the coating. In another embodiment, the pre-hydration occurs due to purposeful leaking of a seal between the expandable member and the outer sheath. In another embodiment, said outer sheath does not tear or come apart during expansion. As explained above and suggested by data in the examples, pre-hydration may help in rapid and complete wetting of the outer sheath as it expands. However, this may be dependent on the formulation of the coating.

In another embodiment, the microporous nature and/or "wettability" of the outer sheath may be distributed over only a portion or portions of the outer sheath. For example, certain locations on the surface of the microporous sheath material may be filled with another material (*e.g.,* silicone and or polyurethane) and made non-microporous and/or non-wettable, but leaving the non-filled areas microporous. Similarly, changes in sheath surface structure (*e.g.,* from "patterning" of the surface) may also be selectively located to create regions of the sheath which are not wettable. Such modifications to the sheath may be useful in instances where therapeutic agents transport through the sheath occur from only certain locations of the sheath. In one embodiment, this approach may be used to deliver therapeutic agents from only a portion of the sheath *e.g.,* to treat only a portion of the radial diameter of a blood vessel which is especially useful where eccentric lesions are present. Such lesions account for approximately 70% of all flow-limiting intravascular lesions. In another embodiment, said distributed wettability can control the rate that said outer sheath becomes wet. Thus, said outer sheath can be modified to have differential permeability throughout the entire outer sheath or can be patterned in such a way to allow for differential permeability at different locations throughout the outer sheath. This embodiment allows for uneven and/or a patterned delivery of therapeutic agents and/or coatings.

In another embodiment, the outer sheath is wet-out by a prescribed preparatory procedure prior to being inserted into the patient. In this embodiment, said agent eluting construct is prewetted in a sterile liquid (*e.g.* saline) supplied with said construct or in the patient's own blood.

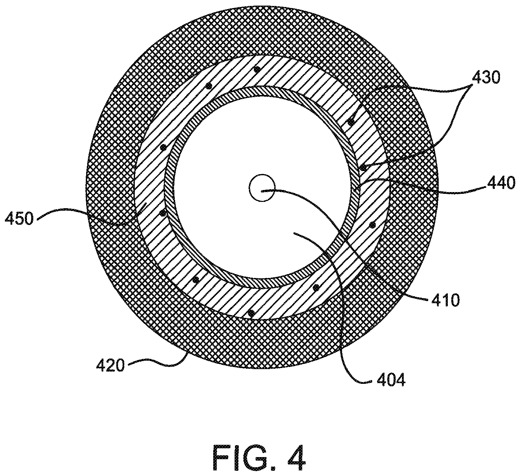

Another embodiment of the invention, as depicted in Figure 4, comprises a cross-section of an agent elution construct in its first, unexpanded, state. In this embodiment, the construct comprises a balloon 404, a substrate or structural layer or cover 440, a hydrophilic coating 450 on balloon 404 and an outer sheath 420. Hydrophilic coating 450 further comprises at least one therapeutic agent 430. Also depicted is guidewire lumen 410 that extends through the length of the balloon. Structural layer 440 can serve many functions. One of its functions may be to serve as a substrate for uniformly applying the hydrophilic coating 450 to the underlying balloon 404. Since some balloon materials may not be conducive to being uniformly coated, the structural layer can serve as a scaffold to achieve a uniform coating. In addition, if the structural layer comprises an elastomer, the structural layer can help with recompaction of the underlying balloon (*see, e.g.,* U.S. Patent 6,120,477, Campbell, et al.). In another embodiment, the structural layer can be coated with said hydrophilic coating and said therapeutic agent prior to placement on an expandable member. With such a pre-fabricated, coating construct, any balloon can be converted to an agent elution construct of the invention. Thus, one embodiment of the invention comprises using a coated structural layer and placing it on any "off the shelf balloon" or OEM balloon to make the balloon a drug delivery balloon. In another embodiment, the hydrophilic coating is coated onto structural layer 440 and then dehydrated or partially dehydrated. In another embodiment, said dehydrated or partially dehydrated hydrophilic coating comprises at least one therapeutic agent. In another embodiment, structural layer 440 and/or outer sheath 420 are wrapped or folded over at a first, uninflated diameter.

A structural layer, for example one made according to the examples below, also provides for a uniform tube to be coated at first state which will concentrically/uniformly expand up to a second state. In contrast, conventional Percutaneous Transluminal Angioplasty (PTA) balloons must be coated at second state (in their molded shape) and then be compacted down to a first state. A structural layer can be coated separate from the catheter or balloon on a mandrel, and later assembled onto the balloon with increased manufacturing yields, lower costs, and higher uniformity. As described above, the coating on said structural layer will be covered by an outer sheath. As the balloon is inflated to its second state, the coating will become hydrated or partially hydrated. The hydrated or partially hydrated coating can flow around said structural layer as the balloon is inflated.

The structural layer can be made from any material that is compatible with the coating and which can be expanded to accommodate expansion of the balloon. These materials include, but are not limited to ePTFE, fluoropolymers, expanded polyethylene, polyvinylchloride, polyurethane, silicone, polyethylene, polypropylene, polyurethane, polyglycolic acid, polyesters, polyamides, elastomers and their mixtures, blends and copolymers, are all suitable. In one embodiment, said structural layer comprises ePTFE. In another embodiment, said ePTFE is imbibed with an elastomer. In another embodiment of the invention, the surface(s) or outward configuration of the structural layer (or expandable member if a structural layer is not used) may be modified with textures, folds, flaps, invaginations, corrugations, protrusions, spikes, scorers, depressions, grooves, pores, coatings, particles, and the like or combinations thereof. In another embodiment, said depressions, grooves, and/or pores can be used increase the effective surface area over which the coating can be placed. This may help in reduction of length or profile of the overall medical device.

In another embodiment of the invention and as an alternative to coating a structural layer which is subsequently combined with an expandable member, the coating material may itself be formed into a structural component that is combined with an expandable member. Such constructs eliminate the requirement for a structural layer per se, yet fully preserve the key functions provided by the coatings of the invention. Such constructs may also improve manufacturability and can be combined with most any expandable member, such as a balloon. A tubular form can be cast or otherwise formed from one or more materials of the described coating and disposed over the balloon prior to placement of the outer sheath. In one embodiment such tubular forms would be made by solvating the coating material(s) into a viscous state and through processes known to the art such as gel extrusion, casting, molding or solution casting/forming formed into the desired tubular shape. The solvent(s) used are subsequently removed to dry or partially dry the tube and makes it easy to dispose over the balloon. During use, the tube is rehydrated much like the coatings used with the invention and described herein.

The outer cover and/or the structural layer can be made from any of the appropriate materials disclosed above. These structures can be made by extrusion or by layering any of the material described above, *e.g.* ePTFE. A layer is considered one thickness of a material which may be wrapped, folded, laid or weaved over, around, beside or under another thickness. A longitudinal pass comprises a distinctive layer or series of layers of material which are wound to form a region or area distinct from surrounding or adjoining parts. For instance, a pass may comprise multiple layers of a material wrapped at a 90° angle relative to the longitudinal axis. This exemplary pass may then be flanked by layers of balloon material wrapped at dissimilar angles in relation to the longitudinal axis, thus defining the boundary of the pass. These layers may be oriented helically, radially or longitudinally. One method for making the structural layer and outer sheath is described below in the examples. In one embodiment, said structural layer and/or outer sheath can vary in thickness along their longitudinal axes. This will allow for different shapes at the second, inflated diameter, and may also vary the amount and/or rate of coating and/or therapeutic agents that are transferred through the outer sheath. In another embodiment, the machine direction of said ePTFE layer is oriented along the longitudinal axis of the medical device. In another embodiment the thickness of the structural layer and/or outer sheath are comprised of different materials to tailor therapeutic agent elution and overall system performance. In another embodiment, the construction of the structural layer and/or outer sheath is discontinuous along the longitudinal axis of the components, *e.g.,* one section of the outer sheath is thicker or comprises a different material, or is thinner than another section. In another embodiment, the ends of the structural layer and/or outer sheath are modified to decrease profile of the agent eluting device at the points on the underlying catheter where the structural layer and/or outer sheath are attached. For example, if the structural layer and/or outer sheath are constructed as tubes, a portion of the circumference of their ends may be skived away to open up the tube, *i.e.,* making the ends of the tube only a portion of their original, full circumference. These end "tabs" are then attached to the catheter (using a method detailed below). Because these tabs comprise less material, the profile at the region of their attachment is decreased. In another embodiment, discrete perforations are created in the outer sheath, further modulating its capacity to elute a coating and/or therapeutic agent.

To make the agent elution construct of the present invention, a hydrophilic layer is formed on an expandable member or a structural layer by applying a hydrophilic substance comprising a therapeutic agent. The hydrophilic layer is applied to the surface of the balloon or a structural layer. The hydrophilic substance may then be optionally bound in place, such as through cross-linking. For a porous surface, the hydrophilic layer may optionally be adsorbed within the porous void spaces of the surface. Certain methods of coating a balloon or structural cover are described in detail in the examples below.

Suitable components for the hydrophilic coating include, but are not limited to, ionic surfactants including benzethonium chloride (*e.g.* HYAMINE®), benzalkonium chloride, cetylpyridinium chloride, cetalkonium chloride, laurtrimonium bromide , myristyltrimethylammonium bromide, cetrimide, cetrimonium bromide, stearalkonium chloride, n,n-diethylnicotinamide, cholesterol, calcium salicylate, methyl salicylate, sodium salicylate, α-tocopherol, thiamine, niacinamide, dimethyl sulfoxide, poloxamers (such as 101, 105, 108, 122, 123, 124, 181, 182, 183, 184, 185, 188, 212, 215, 217, 231, 234, 235, 237, 238, 282, 284, 288, 331, 333, 334, 335, 338, 401, 402, 403, and 407), sorbitan monolaurate, polysorbate 20, polysorbate 40, polysorbate 60, polysorbate 80, polyvinyl alcohol, polyethylene glycol (PEG, molecular weight ranges from 400-50,000, with preferred from 700-15,000), PEG-amine, PEG-modified biopharmaceuticals and/or molecules, PEG amines (that include azido PEG amines and PEG diamines), JEFFAMINES® which are polyoxyalkyleneamines, quartenary ammonium compounds, 1,2-ditetradecanoyl-sn-glycero-3-phosphocholine, 1,2-dimyristoyl-sn-glycero-3-phospho-*rac*-(1-glycerol), 1,2-dimyristoyl-*sn*-glycero-3-phosphocholine, polypropylene glycol, heparin, or heparin derivatives, dextran, agarose, inclusion complexes such as cyclic oligosaccharides like cyclodextrin and its derivatives, including hydroxypropyl-β-cyclodextrin (HPβCD), Captisol® (a trademark of CyDex Pharmaceuticals, Inc.), dimethyl-p-cyclodextrin, α-cyclodextrin (αCD), alginate, polyacrylamide, polyglycidol, poly(vinyl alcohol-co-ethylene), poly(ethyleneglycol-co-propyleneglycol), poly(vinyl acetate-co-vinyl alcohol), poly(tetrafluoroethylene co-vinyl alcohol), poly(acrylonitrile-co-acrylamide), poly (acrylonitrile-co-acrylic acid-co-acrylamide), polyacrylic acid, poly-lysine, polyethyleneimine, polyvinyl pyrrolidone, polyhydroxyethylmethacrylate, cyclodextrins, γ-cyclodextrin, sulfobutylether-β-cyclodextrin, and polysulfone, polysaccharides, and their copolymers, shellolic acid, ipromide, urea, either alone or in combination. Other coatings are known in the art, *see, e.g.,* U.S. Patent Publication 20100233266, can also be used as part of this invention. In another embodiment, said hydrophilic coating is a heparin coating, such those described in U.S. Patents 4,810,784 and 6,559,131.

In another embodiment, hygroscopic substances may be incorporated in the coating to accelerate fluid uptake. These materials include, but are not limited to saccharides, dimethyl sulfoxide, polyvinyl alcohol, glycerol, many salts, including, but not limited to, sodium chloride, zinc chloride, and calcium chloride. Such hygroscopic substances will attract and hold water molecules from the surrounding environment through either absorption or adsorption and help in hydrating said dehydrated coating. Such hygroscopic substances may be combined with any of the excipients described herein and/or commonly known in the art.

Differential Scanning Calorimetry (DSC) can be used to identify and characterize complexes and other physical states of the coating. Fourier Transform Infrared Spectroscopy (FTIR) or Nuclear Magnetic Resonance (NMR) may also be utilized to further characterize complex formation, micelle formation, hydrotrophs, and other formations, which alter the morphology of the therapeutic agent, and to characterize the coating.

A "therapeutic agent" as used herein, which is used interchangeable with the term "drug", is an agent that induces a bioactive response. Such agents include, but are not limited to, cilostazol, everolimus, dicumarol, zotarolimus, carvedilol, anti-thrombotic agents such as heparin, heparin derivatives, urokinase, and dextrophenylalanine proline arginine chloromethylketone; antiinflammatory agents such as dexamethasone, prednisolone, corticosterone, budesonide, estrogen, sulfasalazine and mesalamine, sirolimus and everolimus (and related analogs), anti-neoplastic/antiproliferative/anti-miotic agents such as major taxane domain-binding drugs, such as paclitaxel and analogues thereof, epothilone, discodermolide, docetaxel, paclitaxel protein-bound particles such as ABRAXANE® (ABRAXANE is a registered trademark of ABRAXIS BIOSCIENCE, LLC), paclitaxel complexed with an appropriate cyclodextrin (or cyclodextrin like molecule), rapamycin and analogues thereof, rapamycin (or rapamycin analogs) complexed with an appropriate cyclodextrin (or cyclodextrin like molecule), 17β-estradiol , 17β-estradiol complexed with an appropriate cyclodextrin, dicumarol, dicumarol complexed with an appropriate cyclodextrin, β-lapachone and analogues thereof, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; anesthetic agents such as lidocaine, bupivacaine and ropivacaine; anti-coagulants such as D-Phe-Pro-Arg chloromethyl ketone, an RGD peptide-containing compound, AZX100 a cell peptide that mimics HSP20 (Capstone Therapeutics Corp., USA), heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; vascular cell growth promoters such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; protein kinase and tyrosine kinase inhibitors (*e.g.,* tyrphostins, genistein, quinoxalines); prostacyclin analogs; cholesterol-lowering agents; angiopoietins; antimicrobial agents such as triclosan, cephalosporins, aminoglycosides and nitrofurantoin; cytotoxic agents, cytostatic agents and cell proliferation affectors; vasodilating agents; agents that interfere with endogenous vasoactive mechanisms; inhibitors of leukocyte recruitment, such as monoclonal antibodies; cytokines; hormones or a combination thereof. In one embodiment, said therapeutic agent is a hydrophilic agent. In another embodiment, said therapeutic agent is a hydrophobic agent. In another embodiment, said therapeutic agent is paclitaxel.

In another embodiment of the invention, said coating comprises at least one hydrophilic component that raises the solubility point of a hydrophobic therapeutic agent. As used herein, the term "raises the solubility point of a hydrophobic therapeutic agent" means that there is an increase of concentration of a hydrophobic therapeutic agent at least 10% above the maximum solubility for said therapeutic agent in neat DI-water at room temperature and standard atmospheric conditions. This is usually due to the presence of an additional agent that allows for enhanced solubility (*i.e.,* a hydrophilic component in said coating). This still allows for a portion of the therapeutic agent to not be dissolved into the water. For example, paclitaxel at room temperature in neat DI-water has a solubility limit of about 0.4 µM in water. The addition of hydroxypropyl-β-cyclodextrin at a concentration of 60% (w/v in water) raises the solubilized concentration of paclitaxel in solution to approximately 4 mM, well above a 10% increase in solubility (Sharma et al., Journal of Pharmaceutical Sciences 84, 1223 (1995)).

As used herein, weight percent (wt%) is the dry weight of a coating and/or therapeutic agent after solvent removal. In one embodiment, formulations comprising benzethonium chloride and a hydrophobic agent, such as paclitaxel, the preferred range for said hydrophobic agent are from about 1 wt% to about 70 wt%. In another embodiment, said hydrophobic agent, such as paclitaxel, ranges from about 40 wt% to about 70 wt%. In another embodiment, said hydrophobic agent, such as paclitaxel, ranges from about 20 wt% to about 40 wt%. In another embodiment, said hydrophobic agent, such as paclitaxel, ranges from about 1 wt% to about 20 wt%. In another embodiment, said formulations of benzethonium chloride and a hydrophobic agent, such as paclitaxel, is less than 20 wt% of said hydrophobic agent, such as paclitaxel. In another embodiment, said hydrophobic therapeutic agent is selected from the group consisting of taxane domain-binding drugs, such as paclitaxel, and rapamycin.

In another embodiment, formulations of poloxamer and of a hydrophobic agent, such as paclitaxel, range from about 1 wt% to about 70 wt%, from about 1 wt% to about 50 wt%, from about 1 wt% to about 40 wt%, from about 10 wt% to about 20 wt% of said hydrophobic agent, such as paclitaxel.

In another embodiment, formulations of poloxamer, PEG and of a hydrophobic agent, such as paclitaxel, range from: about 1 wt% to about 70 wt%, about 1 wt% to about 50 wt%, or about 8 wt% to about 40 wt% of a hydrophobic agent, such as paclitaxel; about 1 wt% to about 55 wt%, about 1 wt% to about 40 wt%, or about 5 wt% to about 30 wt% of PEG; and about 1 wt% to about 70 wt%, about 20 wt% to about 70 wt% , about 20 wt% to about 60 wt% of poloxamer, *e.g.* poloxamer-188. In another embodiment, said hydrophobic therapeutic agent is selected from the group consisting of taxane domain-binding drugs, such as paclitaxel, and rapamycin.

In one embodiment, the agent elution construct of the invention comprises a coating comprising benzethonium chloride, and a hydrophobic therapeutic agent, wherein said hydrophobic therapeutic is less than 40 wt% of the dry coating. In another embodiment, said hydrophobic therapeutic agent is about 10 wt% to about 20 wt% of the dry coating and benzethonium chloride is about 80 wt% to about 90 wt% of the dry coating. In another embodiment, said hydrophobic therapeutic agent is selected from the group consisting of taxane domain-binding drugs, such as paclitaxel, and rapamycin.

In another embodiment, the agent elution construct of the invention comprises a coating comprising poloxamer-188, and a hydrophobic therapeutic agent, wherein said hydrophobic therapeutic agent is less than 60 wt% of the dry coating. In another embodiment, said hydrophobic therapeutic agent is about 10 wt% to about 30 wt% of the dry coating and said poloxamer-188 is about 60 wt% to about 75 wt% of the dry coating. In another embodiment, said hydrophobic therapeutic agent is selected from the group consisting of taxane domain-binding drugs, such as paclitaxel, and rapamycin.

In another embodiment, the agent elution construct of the invention comprises a coating comprising poloxamer-188 and PEG, and a hydrophobic therapeutic agent, wherein said hydrophobic therapeutic agent is less than 50 wt% of the dry coating. In another embodiment, said hydrophobic therapeutic agent is less than 50 wt% of the dry coating and PEG is less than 30 wt% of the dry coating. In another embodiment, said hydrophobic therapeutic agent is about 10 wt% to about 30 wt% of the dry coating and PEG is about 10 wt% to about 20 wt of the dry coating. In another embodiment, said hydrophobic therapeutic agent is about 10 wt% to about 20 wt%, PEG is about 10 wt% to about 20 wt%, and poloxamer-188 is about 50 wt% to about 65 wt% of the dry coating. In another embodiment, said hydrophobic therapeutic agent is selected from the group consisting of taxane domain-binding drugs, such as paclitaxel, and rapamycin.

In another embodiment, the agent elution construct of the invention comprises a coating comprising benzethonium chloride and PEG, and a hydrophobic therapeutic agent, wherein said PEG is less than 30 wt% of the dry coating and said hydrophobic therapeutic agent is less than 50 wt% of the dry coating. In another embodiment, said PEG is about 10 wt% to about 20 wt% of the dry coating and said hydrophobic therapeutic agent is about 10 wt% to about 25 wt% of the dry coating. In another embodiment, said PEG is about 10 wt% to about 20 wt% of the dry coating, said hydrophobic therapeutic agent is about 10 wt% to about 25 wt% of the dry coating, and benzethonium chloride is about 50 wt% to about 65 wt% of the dry coating. In another embodiment, said hydrophobic therapeutic agent is selected from the group consisting of taxane domain-binding drugs, such as paclitaxel, and rapamycin.

In another embodiment, the agent elution construct of the invention comprises a coating comprising benzethonium chloride and poloxamer-188, and a hydrophobic therapeutic agent, wherein poloxamer-188 is less than 30 wt% and said hydrophobic therapeutic agent is less than 50 wt% of the dry coating. In another embodiment, poloxamer-188 is about 10 wt% to about 20 wt% of the dry coating and said hydrophobic therapeutic agent is about 10 wt% to about 35 wt% of the dry coating. In another embodiment, said poloxamer-188 is about 10 wt% to about 20 wt%, said hydrophobic therapeutic agent is about 10 wt% to about 25 wt%, and benzethonium chloride is about 50 wt% to about 65 wt% of the dry coating. In another embodiment, said hydrophobic therapeutic agent is selected from the group consisting of taxane domain-binding drugs, such as paclitaxel, and rapamycin.

In another embodiment, the agent elution construct of the invention comprises a coating comprising hydroxypropyl-β-cyclodextrin, and a hydrophobic therapeutic agent, wherein said hydroxypropyl-β-cyclodextrin is equal to or less than 98 wt% of the dry coating. In another embodiment, said hydroxypropyl-β-cyclodextrin is less than 80 wt% of the dry coating. In another embodiment, said hydrophobic therapeutic agent is selected from the group consisting of taxane domain-binding drugs, such as paclitaxel, and rapamycin.

In another embodiment, the agent elution construct of the invention comprises a coating comprising sodium salicylate, and a hydrophobic therapeutic agent, wherein said sodium salicylate is about 75 wt% to about 95 wt% of the dry coating. In another embodiment, said sodium salicylate is less than 80 wt% of the dry coating. In another embodiment, said hydrophobic therapeutic agent is selected from the group consisting of taxane domain-binding drugs, such as paclitaxel, and rapamycin.

The therapeutic agents useful in conjunction with the system of the invention may be delivered to the tissue in various structural forms, including but not limited to micelles, liposomes, micro-aggregates, nanospheres, microspheres, nanoparticles, microparticles, crystallites, inclusion complexes, emulsions, gels, foams, creams, suspensions, and solutions or any combination thereof. In one embodiment, the agent is delivered to the tissue in a solubilized form. In another embodiment, the agent is delivered to the tissue in a gel. In another embodiment, the agent is delivered to the tissue in a solubilized form that precipitates from solution into a solid form. In another embodiment, the agent is delivered to the tissue as a combination of solubilized and solid forms.

The "expandable member" according to the present invention is a balloon catheter which may be expanded, for example *via* application of a torsional or longitudinal force. The latter device can be placed temporarily in any lumen (*e.g.* a vessel) by expanding said device and then removed by collapsing said device by a torsional or longitudinal force. In one embodiment, a structural layer and outer sheath is placed on the device such that when it is expanded, a therapeutic agent will be delivered. In another embodiment, said expandable member allows for blood perfusion to downstream vasculature while implanted in said vessel. This feature may allow for longer implantation durations. In one embodiment, the expandable members may be detached *in vivo,* and optionally retrieved, from placement devices (*e.g.,* catheters). Examples can be found in U.S. Patents 3,996,938, 4,650,466, 5,222,971, and 6,074,339.

In one embodiment, the expandable member is a medical balloon. Balloons useful in the invention may be blow-molded, may be compliant or semi-compliant and may be of various shapes, for example so called "conformable" or "conforming" or "steerable" balloons. In other embodiments, the expandable members may comprise balloons which are constructed of wrapped films, are fiber-wound, are of variable length, are segmented, and/or have controlled inflation profiles. In the latter case, controlled inflation profiles may work to transfer fluid from the exterior of the balloon (or structural layer placed over it) through the sheath in a preferential way. For example a balloon that inflates first in its longitudinal center region, followed by the ends proximal and distal the center region will cause the coating or coating and therapeutic agent to pass through the sheath first in the center region of the sheath. The physical characteristics of said expandable members may also be modified, for example they may have modulus values which differ from one another.

The agent eluting construct of the invention comprises a structural layer and/or the expandable member that comprises a coating (that may or may not comprise at least one therapeutic agent) on said surface of said structural layer and/or the expandable member. Said coating can render said agent eluting construct very rigid. Due to its rigidity said agent eluting construct may be difficult to track through tortuous anatomy. Thus, in one embodiment, after applying coating to said structural layer and/or expandable member, the outer sheath is slipped over said structural layer and/or expandable member and then the coating is cracked by bending and/or twisting said structural layer and/or the expandable member-outer sheath construct. This allows said agent eluting construct to be more conformable, while not allowing any particulates to escape the outer-sheath prior to treatment. In another embodiment, instead of fully coating the structural layer and/or the expandable member, said coating is applied as "rings" of coating such that in between said "rings" of coatings the structural layer and/or the expandable member is conformable and allow said structural layer and/or expandable member to bend at the uncoated region (allows for flexing). Said rings may also reduce hydration time of the coating by maximizing surface area of the coating in contact with a hydrating fluid. Reduced hydration time can improve overall system performance (*e.g.,* time to effect delivery, degree of drug uptake, etc.). In another embodiment, rather than "rings", the coating and/or therapeutic agent are applied to the structural layer and/or the expandable member as an extruded, helically laid-down, continuous beading. In another embodiment, rather than "rings", the coating and/or therapeutic agent are applied to the structural layer and/or the expandable member as discrete dots or other shapes or discrete patterns. In another embodiment, said rings of coating can comprise the same therapeutic agent and/or different therapeutic agent and/or different coatings.

In another embodiment, the coating and/or therapeutic agent are applied to the structural layer and/or the expandable member in a discontinuous fashion. For example, the amount or thickness of coating may be varied over the surface of the substrate. In instances where drug delivery is desired only at the proximal and distal ends of a stent, for example, coatings applied to only the proximal and distal portions of the structural layer, expandable member and/or outer sheath (leaving the middle portion uncoated) may be desirable, especially for treatment or prevention of stent end stenosis. Coating and/or therapeutic agent compounds may similarly vary over the area of the structural layer and/or the expandable member. In another embodiment, the viscosity of the coating and/or therapeutic agent is selected to tailor the rate of drug delivery through the outer sheath.

In another embodiment, said agent eluting construct comprises an underlying medical balloon, a structural layer (optional), a coating comprising a therapeutic agent, and outer sheath wherein said components are mounted on a catheter. In one embodiment, the expanded diameter of said balloon is about 4 mm, about 5 mm, about 6 mm, about 7 mm, about 8 mm, about 9 mm, or about 10 mm in diameter with lengths ranging from about 30 to about 150 mm. In another embodiment, said balloon catheter will range in length from about 90 to about 150 cm. In another embodiment, said eluting balloon of the invention is about 5, 6, 7, 8, 9 or 10 French (Fr) in size before introduction into a body vessel, cavity or duct.

In another embodiment, said agent eluting construct comprises an underlying medical balloon, a structural layer (optional), a coating comprising a therapeutic agent, and outer sheath wherein said components are mounted on a catheter but may be detached from the catheter for short or long term implantation.

According to the present invention said balloon may be formed using any materials known to those of skill in the art. Commonly employed materials include the thermoplastic elastomeric and non-elastomeric polymers and the thermosets.

Examples of suitable materials include but are not limited to, polyolefins, polyesters, polyurethanes, polyamides, polyether block amides, polyimides, polycarbonates, polyphenylene sulfides, polyphenylene oxides, polyethers, silicones, polycarbonates, styrenic polymers, copolymers thereof, and mixtures thereof. Some of these classes are available both as thermosets and as thermoplastic polymers. *See,* U.S. Pat No. 5,500,181, for example. As used herein, the term "copolymer" shall be used to refer to any polymer formed from two or more monomers, *e.g.* 2, 3, 4, 5 and so on and so forth.

Useful polyamides include, but are not limited to, nylon 12, nylon 11, nylon 9, nylon 6/9 and nylon 6/6. The use of such materials is described in U.S. Pat. No. 4,906,244, for example.

Examples of some copolymers of such materials include the polyether-block-amides, available from Elf Atochem North America in Philadelphia, Pa. under the tradename of PEBAX®. Another suitable copolymer is a polyetheresteramide.

Suitable polyester copolymers, include, for example, polyethyelene terephthalate and polybutylene terephthalate, polyester ethers and polyester elastomer copolymers such as those available from DuPont in Wilmington, Del. under the tradename of HYTREL®.

Block copolymer elastomers such as those copolymers having styrene end blocks, and midblocks formed from butadiene, isoprene, ethylene/butylene, ethylene/propene, and so forth may be employed herein. Other styrenic block copolymers include acrylonitrile-styrene and acrylonitrile-butadiene-styrene block copolymers. Also, block copolymers wherein the particular block copolymer thermoplastic elastomers in which the block copolymer is made up of hard segments of a polyester or polyamide and soft segments of polyether may also be employed herein.

Specific examples of polyester/polyether block copolymers are poly(butylene terephthalate)-block-poly(tetramethylene oxide) polymers such as ARNITEL® EM 740, available from DSM Engineering Plastics and HYTREL® polymers available from DuPont de Nemours & Co, already mentioned above.

Suitable materials which can be employed in balloon formation are further described in, for example, U.S. Pat. No. 6,406,457; U.S. Pat. No. 6,284,333; U.S. Pat. No. 6,171,278; U.S. Pat. No. 6,146,356; U.S. Pat. No. 5,951,941; U.S. Pat. No. 5,830,182; U.S. Pat. No. 5,556,383; U.S. Pat. No. 5,447,497; U.S. Pat. No. 5,403,340; U.S. Pat. No. 5,348,538; and U.S. Pat. No. 5,330,428.

The above materials are intended for illustrative purposes only, and not as a limitation on the scope of the present invention. Suitable polymeric materials available for use are vast and too numerous to be listed herein and are known to those of ordinary skill in the art.

Balloon formation may be carried out in any conventional manner using known extrusion, blow molding and other molding techniques. Typically, there are three major steps in the process which include extruding a tubular preform, molding the balloon and annealing the balloon. Depending on the balloon material employed, the preform may be axially stretched before it is blown. Techniques for balloon formation are described in U.S. Pat. No. 4,490,421, RE32,983, RE33,561 and U.S. Pat. No. 5,348,538.

The balloon may be attached to the tubular body by various bonding means known to the skilled artisan. Examples include, but are not limited to, solvent bonding, thermal adhesive bonding and heat shrinking or sealing. The selection of the bonding technique is dependent upon the materials from which the expandable element and tubular body are prepared. Refer to U.S. Pat No. 7,048,713 to Wang for general teachings relating to the bonding of a balloon to a catheter.

The agent eluting constructs provided by the present invention are suitable for a wide range of applications including, for example, a range of medical treatment applications within the body. Exemplary applications include use as a catheter balloon for transferred drug, treating a targeted tissue within the body, and treating any body cavity, space, or hollow organ passage(s) such as blood vessels, the urinary tract, the intestinal tract, nasal cavity, neural sheath, intervertebral regions, bone cavities, esophagus, intrauterine spaces, pancreatic and bile ducts, rectum, and those previously intervened body spaces that have implanted vascular grafts, stents, prosthesis, or other type of medical implants. Additional examples include an agent eluting construct device for the removal of obstructions such as emboli and thrombi from blood vessels, as a dilation device to restore patency to an occluded body passage, as an occlusion device to selectively deliver a means to obstruct or fill a passage or space, and as a centering mechanism for transluminal instruments like catheters. In one embodiment, agent eluting constructs provided by the present invention can be used to treat stent restenosis or treat tissue sites where previously placed drug eluting constructs have failed. In another embodiment, agent eluting constructs as described herein can be used to establish or maintain arteriovenous access sites, *e.g.,* those used during kidney dialysis. In one embodiment, said agent eluting construct comprises a medical balloon and is used for Percutaneous Transluminal Angioplasty (PTA) in patients with obstructive disease of the peripheral arteries. In another embodiment, agent eluting constructs provided by the present invention can be used to treat coronary stenosis or obstructions.

The invention comprises a balloon catheter comprising, a balloon comprising a coating and a therapeutic agent disposed around the outer surface of said balloon, a sheath disposed around said balloon wherein said sheath has a microstructure composed of nodes interconnected by fibrils that prevents macroscopic wetting of said sheath in the unexpanded state, wherein said coating and therapeutic agent are disposed between the surface of the balloon and the sheath, and wherein when said balloon and sheath are expanded, substantially all of said sheath wets out rapidly and allows rapid transfer of said coating through the outer sheath. In one embodiment, said coating is transferred through said outer sheath and onto a target tissue. In another embodiment, said coating remains substantially adhered to the target tissue for greater than 1 minute after balloon deflation. In another embodiment, said sheath undergoes microscopic wetting in a vessel while said balloon and sheath are in the unexpanded state and being delivered to a desired location within a vessel. In another embodiment, bodily fluids substantially wet-out the sheath when said sheath is expanded. In another embodiment, said coating also wets said sheath when said sheath is expanded. In another embodiment, substantially all of said sheath is wet by the time said sheath is fully expanded. In another embodiment, said wetting of the sheath is facilitated when said sheath is in contact with a vessel wall. In another embodiment, said sheath contains a wetting agent to facilitate wetting of the sheath. In another embodiment, said sheath contains the wetting agent polyvinyl alcohol to facilitate wetting of the sheath. In another embodiment, said sheath comprises a fluoropolymer. In another embodiment, said nodes are aligned longitudinally to the longitudinal axis of said balloon catheter and said fibrils are aligned circumferentially to said axis. In another embodiment, said nodes are aligned circumferentially to the longitudinal axis of said balloon catheter and said fibrils are aligned longitudinally to said axis. In another embodiment, said nodes expand (elongate) said outer sheath expands. In another embodiment, said nodes are spread apart as said outer sheath expands. In another embodiment, said fibrils are spread apart as said outer sheath expands. In another embodiment, said sheath comprises ePTFE. In another embodiment, said coating comprises a hydrophilic component. In another embodiment, said coating comprises at least one hydrophilic component selected from the group consisting of benzethonium chloride, PEG, poloxamer, sodium salicylate, and hydroxypropyl-β-cyclodextrin. In another embodiment, said therapeutic agent is a hydrophilic agent. In another embodiment, said therapeutic agent is a hydrophobic agent. In another embodiment, said hydrophobic agent is selected from the group consisting of taxane domain-binding drugs, such as paclitaxel, and rapamycin. In another embodiment, said balloon further comprises a structural layer. In another embodiment, said structural layer comprises said coating and therapeutic agent. The microstructure of the sheath changes as said balloon expands.

Other embodiments of the invention comprise a method of delivering a therapeutic agent to a desired location within a vessel comprising, inserting a catheter in a vessel, said catheter comprising an expandable member comprising a hydrophilic coating with a therapeutic agent, a sheath disposed around said expandable member, wherein said sheath has a variably permeable microstructure that substantially prevents transfer of said coating and said therapeutic agent through said sheath in the unexpanded state, and wherein said coating and therapeutic agent are disposed between the surface of the expandable member and the sheath, advancing said catheter to a desired location within said vessel, and expanding the expandable member and sheath at the desired location within said vessel, and wherein substantially all of said expanded sheath allows transfer of said coating and therapeutic agent from between the surface of the expandable member and the sheath to an area external to said sheath while preventing transfer of particles out of said sheath greater than about 25 microns in size. In one embodiment, said expandable member is a medical balloon. In another embodiment, said sheath rapidly wets out during expansion and allows rapid transfer of said coating and therapeutic agent. In another embodiment, said sheath undergoes microscopic wetting in a vessel while said balloon and sheath are in the unexpanded state and being delivered to a desired location within a vessel. In another embodiment, said wetting of the sheath is facilitated when said sheath is in contact with the vessel wall. In another embodiment, said sheath contains a wetting agent to facilitate wetting of the sheath. In another embodiment, said sheath contains the wetting agent polyvinyl alcohol to facilitate wetting of the sheath. In another embodiment, said sheath comprises a fluoropolymer. In another embodiment, the sheath comprises a microstructure comprised of nodes interconnected by fibrils. In another embodiment, said nodes are aligned longitudinally to the longitudinal axis of said balloon catheter and said fibrils are aligned circumferentially to said axis. In another embodiment, said nodes are aligned circumferentially to the longitudinal axis of said balloon catheter and said fibrils are aligned longitudinally to said axis. In another embodiment, said nodes expand (elongate) said outer sheath expands. In another embodiment, said nodes and are spread apart as said outer sheath expands. In another embodiment, said fibrils are spread apart as said outer sheath expands. In another embodiment, said sheath comprises ePTFE. In another embodiment, said therapeutic agent is a hydrophilic agent. In another embodiment, said therapeutic agent is a hydrophobic agent. In another embodiment, said hydrophobic agent is selected from the group consisting of taxane domain-binding drugs, such as paclitaxel, and rapamycin. In another embodiment, said coating comprises at least one hydrophilic component selected from the group consisting of benzethonium chloride, PEG, poloxamer, sodium salicylate, and hydroxypropyl-β-cyclodextrin. In another embodiment, said expandable member further comprises a structural layer. In another embodiment, said structural layer comprises said coating and therapeutic agent. The microstructure of the sheath changes as said expandable member expands.

In another embodiment of the invention, agent elution constructs of the invention can be applied in configurations other than those which are radially circular. For example, this invention can be used in conjunction with planar devices such as wound dressings, implantable patches (including vascular and hernia patches), transdermal patches, filters, various device delivery components, occluders, and orthopedic implants. In one embodiment, the system of the invention may be incorporated into an implantable lead (*e.g.,* a cardiac or neurostimulation lead), provided the lead is compatible with an expandable member, *e.g.,* features a lumen or pocket into which an expandable member is positionable.

In another embodiment, the expandable medical device of the invention is combined with an occlusion device such as a balloon located proximate the device. Said occlusion device may mitigate the movement of drug far from the treatment site. In one embodiment, the bodily fluids isolated by this system may be withdrawn from the body by aspiration prior to removal of the system.

Although not part of the present invention, a kit comprising a structural layer comprising a dehydrated or partially dehydrated coating (further comprising a therapeutic agent) and an outer sheath over said structural layer is here disclosed. Such a kit can convert an off the shelf balloon catheter into an agent eluting construct of the invention. Said kit comprises an adhesive (including tapes and liquid adhesives) for bonding said structural layer and outer sheath to a balloon catheter. Said structural layer, outer sheath and adhesive are sterile, placed in a container with an instruction pamphlet explaining how to apply said structural layer and outer sheath onto said balloon catheter. In another embodiment, said balloon catheter is also sterile.

Another embodiment the invention comprises a balloon catheter comprising a mass transport barrier and a solubilized therapeutic agent, wherein said mass transport barrier has a first configuration that is substantially permeable to bodily fluids and impermeable to the solubilized therapeutic agent and a second configuration, that is substantially permeable to the solubilized therapeutic agent but impermeable to particles greater than about 25 µm. In one embodiment, said a mass transport barrier is treated with a wetting agent, as described above.

Another embodiment the invention comprises a method of delivering a bioactive agent to biological target through a mass transport barrier, said method comprising a mass transport barrier and a solubilized therapeutic agent, wherein said mass transport barrier has a first configuration that is substantially permeable to bodily fluids and impermeable to the solubilized therapeutic agent and a second configuration that is substantially permeable to the solubilized therapeutic agent but impermeable to particles greater than about 25 µm, wherein upon of an application of mechanical force to the mass transport barrier induces the change between the first and second configurations thereby allowing controlled permeation of the solubilized therapeutic agent through the mass transport barrier. In one embodiment, said a mass transport barrier is treated with a wetting agent, as described above.

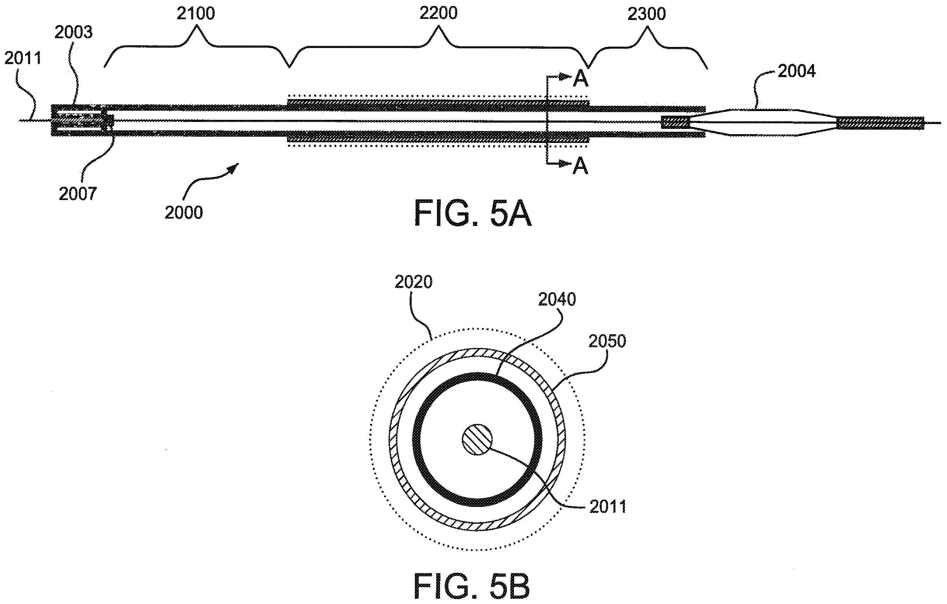

Due to the toxicity of some of the drugs delivered, it is important to deliver therapeutic agents to a specific target. In addition, if several areas are to be targeted for therapeutic agent delivery, the problem of overlapping treatment (*i.e.,* areas that may get several doses of a therapeutic agent) and the need to swap multiple drug eluting balloon catheters can be of major concern. One way to overcome these deficiencies is shown in Figures 5A and B. Figure 5A illustrates a catheter that can be tracked to a targeted area and also be expanded by an expandable device, such as a medical balloon. Catheter 2000 comprises tip 2003 that interfaces with guidewire 2011. Guidewire 2011 may further comprise guidewire stop 2007. Guidewire stop 2007 can engage with tip 2003 and allow the catheter to be tensioned for better balloon tracking. Catheter 2000 further comprises uncoated section 2100, a coated section 2200, and a stiffer tube section 2300. Figure 5A further depicts a balloon catheter with a balloon 2004 at the distal end of said balloon catheter. Said balloon catheter with balloon 2004 can be placed inside said catheter 2000. Stiffer tube section 2300 allows for said balloon catheter to be more easily inserted into catheter 2000.

Figure 5B depicts a cross section at line A-A of coated section 2200. Figure 5B depicts a distensible layer 2040 (similar to the structural layer described above), a coating (comprising a therapeutic agent) 2050, outer sheath 2020 and guidewire 2011.

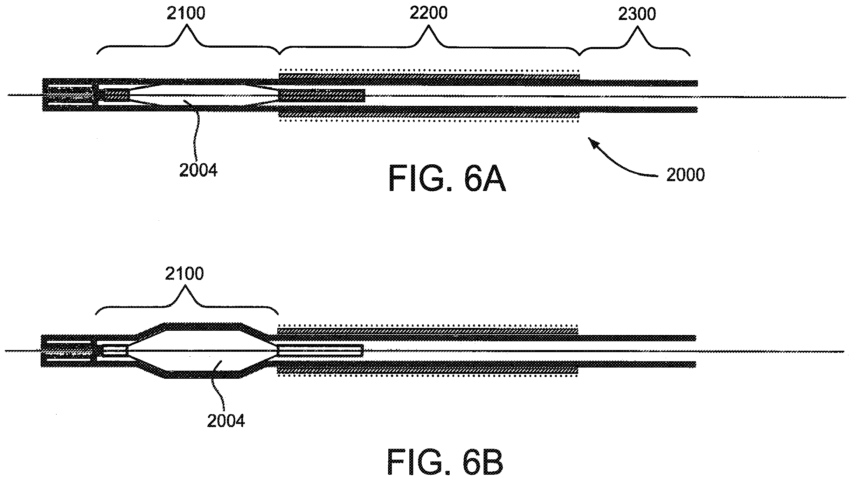

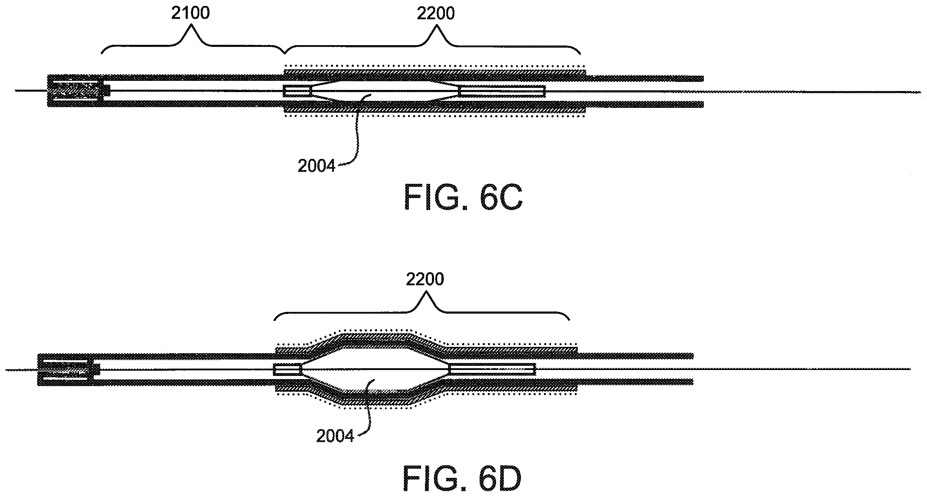

Figures 6A through 6D depict the procedural steps for one method of use employing this embodiment. Catheter 2000 is tracked and placed in a targeted vessel for treatment. Then balloon 2004 is tracked into catheter 2000 to a desired location within catheter 2000, as depicted in Figure 6A. In one embodiment, balloon 2004 is tracked and inflated in uncoated section 2100 to deliver a standard Percutaneous Transluminal Angioplasty (PTA) treatment, as depicted in Figure 6B. Then, balloon 2004 is deflated after PTA, catheter 2000 is advanced distally to position coated section 2200 at the PTA site and balloon 2004 is repositioned under coated section 2200, as depicted in Figure 6C. Then, balloon is inflated in coated section 2200, as depicting in Figure 6D. This will facilitate delivery of a therapeutic agent and/or coating to the vessel. In another embodiment, said balloon is deflated, repositioned to another area of coated section to deliver another dose of a therapeutic agent. In another embodiment, to aid visualization by the clinician, radiopaque or other imaging markers are incorporated in catheter 2000 and/or balloon catheter 2004. In another embodiment, several doses can be delivered to different areas in a vessel by repositioning balloon 2004 and/or catheter 2000. The mechanisms by which the catheter is made, the coating and therapeutic agent are loaded and delivered are described above. In another embodiment, said catheter comprises an elastomeric element (as described above) so that after balloon inflation catheter 2000 can recompact to or near to its delivery diameter.

Thus, one embodiment of the invention comprises a system of delivering a therapeutic agent comprising, a catheter comprising a distensible layer, a coating comprising a therapeutic agent disposed around said distensible layer, and an outer sheath over said distensible layer and said coating; wherein said outer sheath has a variably permeable microstructure that prevents unintended transfer of therapeutic agent through said outer sheath, a medical balloon catheter, wherein said medical balloon is on the distal end of a catheter; wherein said medical balloon can be placed with said catheter; and wherein when said medical balloon is inflated in said catheter, it will distend said distensible layer and outer sheath allowing rapid transfer of said coating and therapeutic agent to an area external to said outer sheath. In one embodiment, said outer sheath prevents the transfer of particles out of said sheath greater than about 25 microns in size. In another embodiment, said outer sheath rapidly wets out during expansion and allows rapid transfer of said coating and therapeutic agent. In another embodiment, said sheath undergoes microscopic wetting in a vessel while said balloon and sheath are in the unexpanded state and being delivered to a desired location within a vessel. In another embodiment, said sheath comprises a wetting agent and will wet out completely when in contact with fluid in a first diameter. In another embodiment, said coating hydrates when said outer sheath is in a first diameter. In another embodiment, said outer sheath comprises a fluoropolymer. In another embodiment, said outer sheath comprises ePTFE. In another embodiment, said hydrophobic agent is selected from the group consisting of taxane domain-binding drugs, such as paclitaxel, and rapamycin. In another embodiment, said coating comprises at least one hydrophilic component selected from the group consisting of benzethonium chloride, PEG, poloxamer, sodium salicylate, and hydroxypropyl-β-cyclodextrin.

While particular embodiments of the present invention have been illustrated and described herein, the present invention should not be limited to such illustrations and descriptions. It should be apparent that changes and modifications may be incorporated and embodied as part of the present invention within the scope of the following claims. The following examples are further offered to illustrate the present invention.

### EXAMPLES

### Example 1: Preparation of a Structural Cover

A structural cover was prepared using methods as essentially taught in U.S. Patent 6,120,477 (Campbell, et al.). A film tube was made by helically wrapping 20 layers of a highly fibrillated 5 micron thick ePTFE film (U.S. Patent 5,476,589 to Bacino) at an 83.4° angle to the tubular axis on a 7 mm stainless steel mandrel. Ten layers of the ePTFE were wrapped in one direction and ten layers were wrapped in the opposing direction. The mandrel was baked in an oven set at 380° C for 6 minutes to fuse the layers together. The resulting tube was removed from the mandrel and "necked" (stretched) down to a diameter below 2.2 mm. This necked tube was placed onto a 2.2 mm stainless steel mandrel and overwrapped with approximately 5 layers of a sacrificial ePTFE film to prevent the tube from wrinkling in the subsequent steps. Next, the tube construct was uniformly compressed to approximately 65% of its original length. The construct was placed in an oven set at the 380° C for 1 minute and then the sacrificial ePTFE layer was removed. This construct was removed from the mandrel and cut to a 65.0 mm length. In alternate embodiments, this structural layer may comprise an elastomer to aid in recompaction of the underlying balloon (*see, e.g.,* U.S. Patent 6,120,477, Campbell, et al.).

### Example 2: Assembly of a Structural Cover onto a Balloon Catheter

A semicompliant balloon catheter was purchased from Bavaria Medizin Technologie, Oberpfaffenhofen, Germany (model # BMT-035, article# 08PL-604A, with balloon dimensions of 6.0 mm x 40 mm). The balloon has the following specifications: a nylon balloon with a 6 atmosphere (atm) nominal inflation pressure and a 14 atm rated burst pressure, a 6 mm nominal diameter, 40 mm balloon working length, mounted on a 0.9 mm guidewire compatible catheter.

The structural tube, as described in Example 1, was centered over the semicompliant balloon and the ends were wetted with a Loctite 7701 primer (Henkel AG & Co. KgaA, Düsseldorf, Germany). The ends were then fixedly attached to the catheter using five layers of a 6.4 mm width of ePTFE film which were wrapped circumferentially around the balloon ends while Loctite 4981 (Henkel AG & Co. KgaA, Düsseldorf, Germany) was applied to the film.

The structural cover was colored black using a Sharpie® permanent marker (Sanford Corporation, Oak Brook, IL). The coloring of the structural cover was used to show the extent of outer sheath wetting, as described in more detail below. The structural tube is also known herein as the "structural cover", especially when it is placed and secured over a balloon.

### Example 3: Application of a Hydrophilic Coating to a Structural Cover

A 5% (by weight) aqueous solution of polyvinyl alcohol (PVA, USP grade, Spectrum Chemicals & Laboratory Products, Gardena, CA) was prepared. This solution is referred herein as Solution 3. A structural tube was assembled onto a balloon catheter as described in Example 2, and was dip-coated with Solution 3 for 30 seconds while rotating. After the 30 seconds, the device was removed from Solution 3. While rotating the device, a heat gun was used to blow warm air (of about 40° C) over the device for approximately 3 minutes. This process was then repeated two additional times. Next, the device was placed into an oven set at 60° C for approximately 10 minutes.

The resulting coated structure had an outer diameter (OD) of less than 3.2 mm.

### Example 4: Preparation of an Outer Sheath

An outer sheath layer was prepared using the following method. A film tube was created by helically wrapping four layers of a thin ePTFE film (as described in U.S. 5,814,405 Branca et al.) at a 75° angle to the tubular axis on a 6 mm stainless steel mandrel. Two layers of the ePTFE were wrapped in one direction and two layers are wrapped in the opposing direction. The mandrel comprising the ePTFE layers was baked in an oven set at 380° C for 6 minutes to fuse the layers together. The resulting tube was removed from the mandrel and necked down to a diameter below 3.2 mm. This necked tube was stretched up by slipping the tube onto a 3.2 mm stainless steel mandrel. The tube was then overwrapped with approximately five layers of a sacrificial ePTFE film to prevent wrinkling during the subsequent step. Next, the tube construct was uniformly compressed to approximately 90% of its original length. The construct was then placed in an oven set at 380° C for 1 minute. After baking the construct, the sacrificial ePTFE layer was removed. The tube construct was then removed from the mandrel and cut to a 65 mm length to form the outer sheath layer.

### Example 5: Assembly of an Outer ePTFE Sheath onto a Coated Balloon Catheter

The outer sheath layer, as prepared in Example 4, was then centered over the coated section of the balloon described in Example 3 and the ends were wetted with a Loctite 7701 primer (Henkel AG & Co. KgaA, Düsseldorf, Germany). The ends of the outer sheath layer were then fixedly attached to the balloon using five layers of a 6.4 mm width of ePTFE film. Specifically, the ePTFE film layers were wrapped circumferentially around the balloon ends while Loctite 4981 (Henkel AG & Co. KgaA, Düsseldorf, Germany) was applied to the film.

### Example 6: Assembly of an Outer Sheath onto an Uncoated Balloon Catheter

The outer sheath layer, as prepared in Example 4, was centered over the uncoated section of the balloon described in Example 2 and the ends were wetted with a Loctite 7701 primer (Henkel AG & Co. KgaA, Düsseldorf, Germany). The ends of the outer sheath layer were then fixedly attached to the balloon using five layers of a 6.4 mm width of ePTFE film. Specifically, the ePTFE film layers were wrapped circumferentially around the balloon ends while Loctite 4981 (Henkel AG & Co. KgaA, Düsseldorf, Germany) was applied to the film.

### Example 7: Methods for Characterizing In Vitro Wetting of Balloon Catheters in Blood

As described above, wetting is the displacement of air by a fluid in an ePTFE structure. It is known to those skilled in the art that ePTFE that is not wet by a fluid is white or opaque in appearance. It is also known to those skilled in the art that ePTFE that is macroscopically wet by a fluid is translucent in appearance. Accordingly, if the outer sheath of a balloon catheter, as prepared in Example 4, has been wet by blood, or another fluid, the outer sheath will become translucent and the underlying structural cover (previously colored black, see Example 2) will become visible.

The test methods described below were used to test wetting of the balloon cover. Specifically, the test described below was used to determine the degree of wetting of the outer sheath of an agent eluting construct after placement in blood at the construct's first state (unexpanded state) and the degree of wetting of the outer sheath after pressurization (expanded state) and contact with a mock vessel wall.

Blood was harvested from a canine, citrated to prevent clotting, and placed into a 50 ml vial. A balloon catheter construct was fully submerged in the canine citrated blood in its deflated state (first state) for 20 minutes. After 20 minutes, the balloon was removed and fully rinsed with saline.

The balloon construct was visually inspected for signs of wetting of the outer sheath. Pictures were taken, and results were noted as "degree of wetting at first state". Visual signs of sheath wetting include the appearance of black regions along the balloon. These black regions become apparent as the outer sheath wets and becomes translucent, allowing for visualization of the underlying black structural cover. A subjective rating scale was used to designate the degree of wetting where a completely wet sheath would be a '10' and fully non-wet sheath would be a '0'. Partial wetting earned a rank correlating to the degree of wetting.

After ranking, the same balloon catheter was placed into a 5.9 mm diameter rigid tube (70 mm in length) submerged in the canine citrated blood. The balloon catheter (which has a nominal inflation diameter of 6 mm) was then inflated to 6 atm for 1 minute. At this pressure, the balloon catheter achieved full apposition against the rigid tube's wall. After a 1 minute inflation period, the balloon catheter was deflated, removed from the tube, and rinsed with saline. After rinsing, the balloon catheter was photographed, and re-inflated to 6 atm and visually inspected.

Pictures were taken, and results were noted as degree of wetting at 6 atm inflation as described above.

The balloon catheter was then reinserted into the 5.9 mm diameter rigid tube (70 mm in length) submerged in the canine citrated blood. The balloon catheter (which has a nominal inflation diameter of 6 mm) was then inflated to 12 atm for 1 minute. At this pressure, the balloon catheter achieved full apposition against the rigid tube's wall. After the 1 minute inflation period, the balloon catheter was deflated, removed from the tube, and rinsed with saline. After rinsing, the balloon catheter was photographed, re-inflated to 12 atm and visually inspected.

Pictures were taken, and results were noted as degree of wetting at 12 atm inflation.

### Example 8: Effect of Hydrophilic Coating on Outer Sheath Wetting in Blood with Vessel Contact

A PVA coating was applied to a structural cover on a balloon catheter (as described in Example 3). This balloon catheter is herein referred to as Device 8a. The structural cover of the second balloon catheter (herein referred to as Device 8b) was left uncoated.

Outer sheaths were prepared as described in Example 4. An outer sheath was then assembled onto Device 8a as described in Example 5. An outer sheath was then assembled onto Device 8b as described in Example 6.

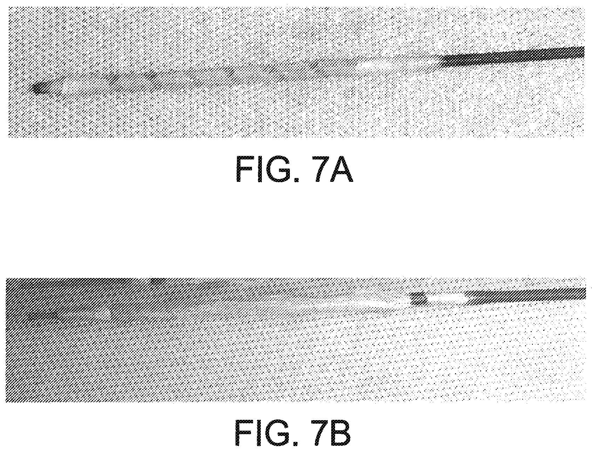

Devices 8a and 8b underwent testing for *in vitro* blood wetting according to the methods described in Example 7. The results of this experiment are detailed in Table 1 and Figures 7 through 9.

**Table 1: Degree of Wetting of Balloon Catheters with and without a Hydrophilic Coating**

| **Degree of Wetting** | **Device 8a (with hydrophilic coating)** | **Device 8b (without hydrophilic coating)** |
|---|---|---|
| at first state | 1 | 1 |
| at 6 atm inflation | 5 | 2 |
| at 12 atm inflation | 10 | 3 |

As shown in Figure 7 and in Table 1, when Device 8a (Figure 7A) and Device 8b (Figure 7B) were submerged in blood in an unexpanded state, the outer sheaths of these devices did not substantially wet and did not become translucent. Therefore, the colored (black) structural cover below the outer sheath was not visible through the outer sheath.

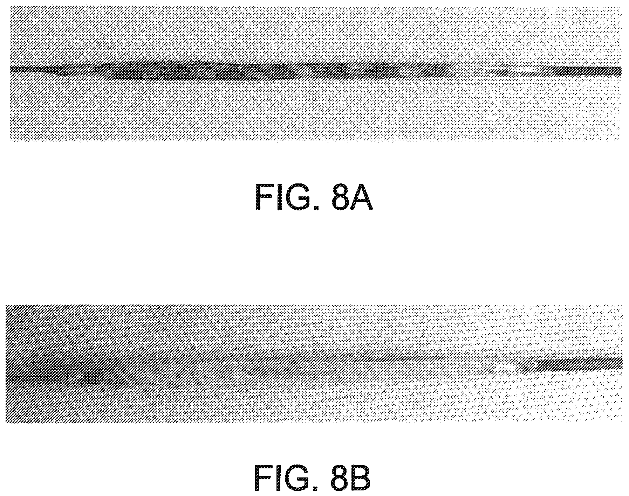

As shown in Figure 8 and in Table 1, when Device 8a (Figure 8A) and Device 8b (Figure 8B) were submerged in blood and expanded to a pressure of 6 atm (as described above), the outer sheath on Device 8a underwent substantial wetting whereas the outer sheath on Device 8b was only partially wetted.

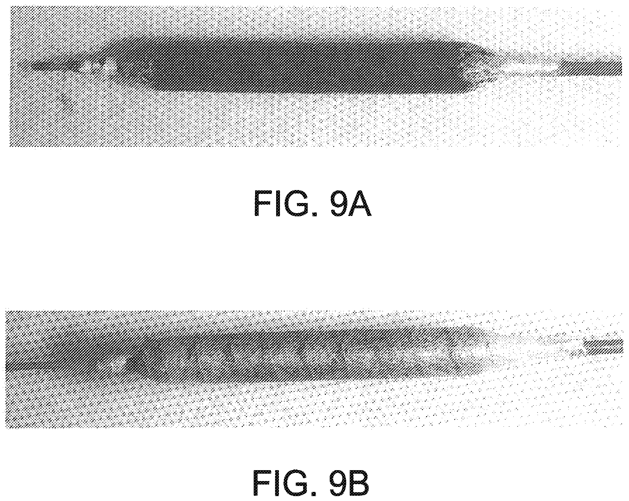

As shown in Table 1, when Device 8a (Figure 9A) and Device 8b (Figure 9B) were submerged in blood and expanded to a pressure of 12 atm (as described above), the outer sheath of Device 8a underwent complete wetting whereas the outer sheath of Device 8b was incompletely wet. Thus, these data suggest that the hydrophilic coating of Device 8a aids in rapid cover wetting.

### Example 9: Effect of Vessel Contact on the Extent of In Vitro Balloon Catheter Wetting

The experiment described herein was used to determine the effect of vessel contact on balloon catheter wetting.

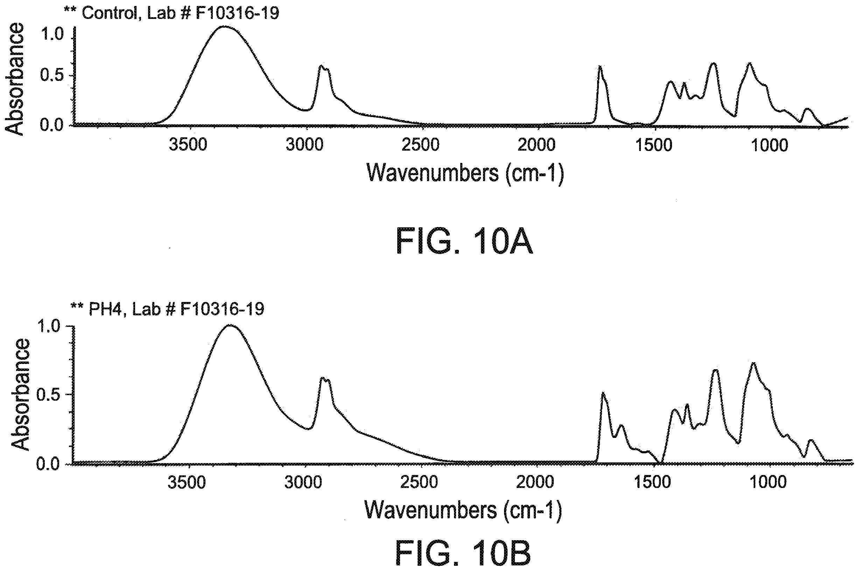

A coating containing PVA (*i.e.,* a hydrophilic coating) was applied to a structural cover (as described in Example 3). A sample of the solution used in the coating process was analyzed by Fourier Transform Infrared Spectroscopy (FTIR). Figure 10A is the interferogram of this analysis. An outer sheath (as prepared in Example 4) was placed onto the balloon catheter (as described in Example 5). This balloon catheter construct is herein referred to as Device 9. Device 9 underwent testing for *in vitro* blood wetting according to the method described below.

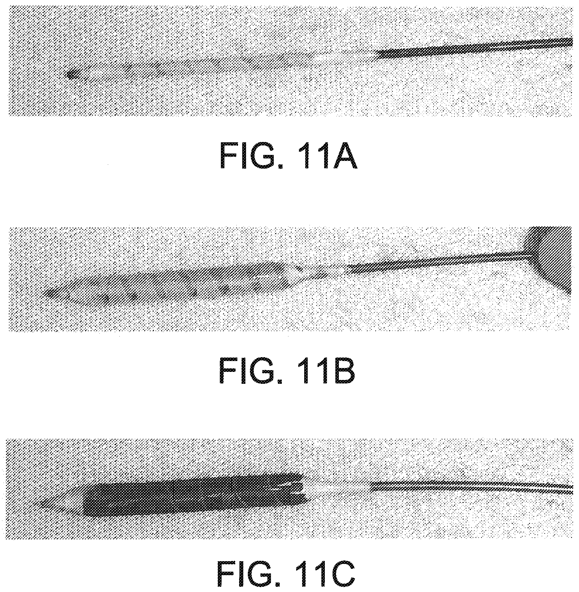

Blood was harvested from a canine, citrated to prevent clotting, and placed in a 50 ml vial. Device 9 was fully submerged in the blood at first state (unexpanded) for 20 minutes. After 20 minutes, Device 9 was removed from the blood, fully rinsed with saline, and photographed (Figure 11A)

Device 9 (which has a nominal inflation diameter of 6 mm) was again submerged in the blood and was inflated to 12 atm for 1 minute. After the 1 minute inflation period, Device 9 was deflated, removed from the blood, and rinsed with saline. After rinsing, Device 9 was re-inflated to 12 atm, visually inspected, and photographed (Figure 11B). Device 9 was then deflated.

Next, Device 9 was inserted into a 5.9 mm diameter rigid tube (70 mm in length) that was submerged in the canine blood. Device 9 was re-inflated to 12 atm for 1 minute. At this pressure, Device 9 achieved full apposition to the tube's wall. After the 1 minute inflation period, Device 9 was deflated, removed from the blood, and rinsed with saline. After rinsing, Device 9 was re-inflated to 12 atm, visually inspected, and photographed (Figures 11C). At this time a glass microscope slide was wiped across the outermost surface of Device 9 to collect any coating that had migrated through the outer sheath. The microscope slide was analyzed by FTIR, Fourier Transform Infrared Spectroscopy. Figure 10B is the interferogram of this analysis. Comparing Figures 10A and 10B, the data suggests that PVA from the coating on Device 9 was transported through the outer sheath upon inflation.

As shown in Figures 11A through 11C, the outer sheath of Device 9 underwent more complete blood wetting after contact with the rigid tube, as depicted in Figure 11C.

### Example 10: Effect of an Outer Sheath on Coating Particulation from a Balloon Catheter

The experiment described here characterizes particulation from coated balloon catheters assembled with and without an outer sheath over the coating.

Four structural covers were prepared as described in Example 1. Each structural cover was separately assembled onto a different balloon catheter (as described in Example 2). The structural covers of the four balloon catheters where coated by the method described below.

A 5% (by weight) aqueous solution of PVA (USP grade, Spectrum Chemicals & Laboratory Products, Gardena, CA) was prepared. This solution is herein referred to as Solution 10.

Next, the following additives were added to 16.3 g of Solution 10: 3.0 g hydroxypropyl-β-cyclodextrin (Sigma-Aldrich, St. Louis, MO), 0.3 g of 2 µm polystyrene microspheres (Polysciences, Warrington, PA), 0.3 g of 5 µm polystyrene microspheres (Polysciences, Warrington, PA), 0.9 g of 10 µm polystyrene microspheres (Polysciences, Warrington, PA), and 0.9 g of 25 µm polystyrene microspheres (Polysciences, Warrington, PA). This resulting coating formulation is herein referred to as Formulation 10B.

Next, the balloon catheters with assembled structural covers were dipped into Formulation 10B for 30 seconds while rotating. After the 30 seconds, the devices were removed from Formulation 10B. While rotating the devices, a heat gun was used to blow warm air (about 40° C) over the devices for approximately 3 minutes. This process was then repeated two additional times. Then the devices were placed into an oven set at 60° C for approximately 10 minutes.

After coating, two of the balloon catheters were not fit with an outer sheath. These coated, sheath-less, balloon catheters are herein defined as Devices 11C and 11D.

After coating, the other two balloon catheters were fit with outer sheaths. Specifically, two separate outer sheaths were prepared according to Example 4. Then each outer sheath was centered over the coated section of the balloon catheter and the ends were wetted with a Loctite 7701 primer. The ends were then fixedly attached using a reinforcing film wrap. The film wrap comprised five layers of a 6.4 mm width of ePTFE film which were wrapped circumferentially around the balloon ends while Loctite 4981 was applied to the film. The resulting coated balloon catheters with attached outer sheath are herein defined as Devices 11e and 11f.

Next, all four devices were subjected to particulation testing utilizing the method described below.

A 25% (by weight) solution of isopropyl alcohol in water was passed through a 0.2 µm filter and collected in a clean 100 ml graduated glass cylinder. This solution is herein referred to as the collection media. The test device was placed in the graduated cylinder so that the balloon was submerged in the collection media. The device was then immediately inflated to 6 atm for 1 minute. After this time, the device was deflated and immediately removed from the graduated cylinder. Particles in the collection media were then analyzed by an Accusizer Particle Sizer (780/SIS PSS NICOMP, Santa Barbara, CA USA) according to test method described by United States Pharmacopeia (USP) monograph 788 for small volume injectables.

As described above two treatment groups were analyzed with a sample size of two per treatment. The treatment groups were: Coated, sheath-less balloon catheters (Devices 11c and 11d); Coated balloon catheters with attached outer sheaths (Devices 11e and 11f).

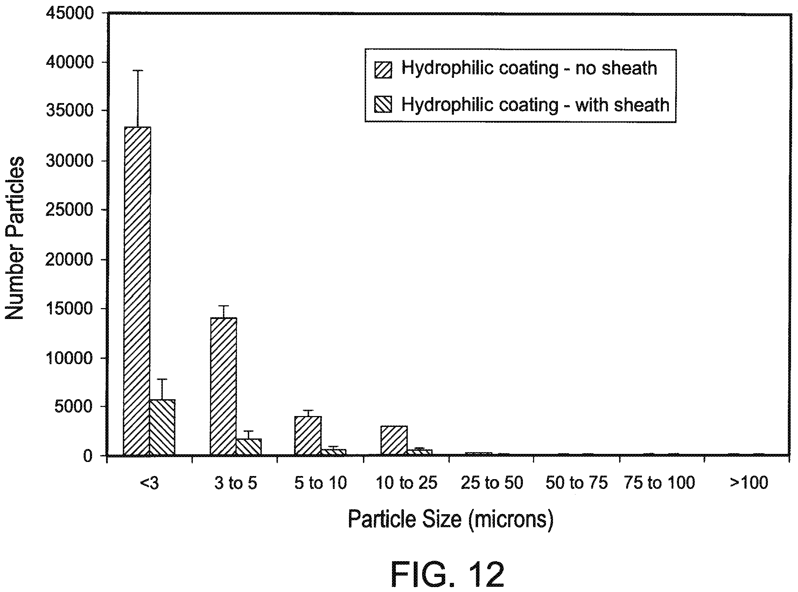

These data are summarized in Figure 12 as mean particle distributions for the three treatment groups. As shown, the outer sheath reduces particulation of the coated devices.

### Example 11: Application of a Texas Red Coating to a Structural Cover

A 5% (w/v) aqueous solution of PVA (USP grade, P1180, Spectrum Chemicals & Laboratory Products, Gardena, CA) was prepared. Then, 0.0833 g of dextran (101509, MP Biomedicals, Solon, OH) was added to 5 ml of the 5% (w/v) PVA solution. This solution is herein referred to as Solution 11b. Next, 10 mg of Texas-red-labeled-dextran (D3328, Invitrogen, Carlsbad, CA) was added to 2 g of the PVA/dextran solution. This solution is herein known as Solution 11c. Solution 11c was vortexed for approximately one minute.

A structural cover was prepared as described in Example 1 and then assembled onto a balloon catheter as described in Example 2. This device was then coated with Solution 11c according to the method described below.

Approximately 0.33 ml of Solution 11c was applied to the device while rotating. The device was then allowed to dry for 10 minutes under warm air. This process was then repeated two additional times. Then the device was allowed to dry overnight at 40° C.

### Example 12: Delivery of Texas-Red-Labeled-Dextran to an Explanted Vessel from a Coated Balloon Catheter

A cryoprotectant solution was prepared by mixing 100 ml of bovine serum (35022-CV, Mediatech, Manassas, VA) with 12.8 ml of DMSO (D-8779, Sigma, St. Louis, MO) and 3.86 g of sucrose (S3928, Sigma, St. Louis, MO). Two segments of canine carotid artery were harvested and placed into separate vials containing the cryoprotectant solution. The vials were stored at -20° C until the time of testing.

A structural cover (as prepared in Example 1) was assembled onto a balloon catheter (as described in Example 2). A hydrophilic coating was then applied to the balloon catheter as described in Example 11. As noted in Example 11, this coating contained a fluorescent molecule (Texas-red-labeled-dextran). An outer sheath layer (previously prepared per Example 4) was then assembled onto the coated balloon catheter (per Example 5). This balloon catheter is herein referred to as Device 12.

At the time of testing, one of the vials containing a segment of cryopreserved artery was thawed. The artery was removed from the vial and submerged in heparinized canine blood (37° C).

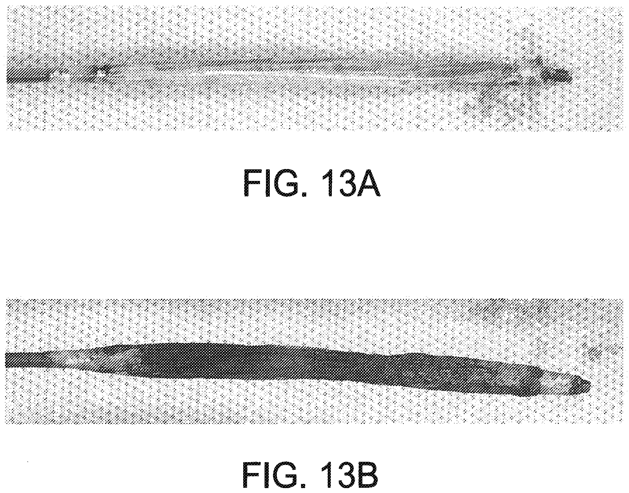

Device 12 was placed in heparinized canine blood (37° C) for 5 minutes. After the 5 minutes, Device 12 was not wet-out and was photographed (Figure 13A) after rinsing with saline.

Then, Device 12 was inserted into the artery and inflated to 6 atm for 1 min. Device 12 was deflated, removed from the artery, and photographed (Figure 13B). After the 1 minute inflation, Device 12 was observed to have wet-out. The artery was rinsed with 25 ml of heparinized canine blood for 5 minutes. Then the artery was incubated in another 25 ml of heparinized canine blood for 5 minutes. Afterward, the artery was then placed in a buffered formalin solution (10% Neutral Buffered Formalin, VWR, Cat# BDH0502-20L, West Chester PA) for fixation and storage. This artery herein defined as the Test Artery.

The second vial containing a segment of cryopreserved artery was thawed. This artery was removed from the vial and placed in a buffered formalin solution (10% Neutral Buffered Formalin, VWR, Cat# BDH0502-20L, West Chester PA) for fixation and storage. This artery served as a control arterial segment and had no contact with Device 12. This artery herein defined as the Control Artery.

The Test and Control Arteries were each separately cut into approximately 1 cm samples and placed into OCT compound (4583, Tissue-Tek, Sakura Finetek, Torrance, CA). The Test and Control Artery samples were frozen in an isopentane/liquid nitrogen solution (2-Methylbutane, M32631-4L, Sigma Aldrich, Saint Louis, MO)

While frozen, a cryostat was used to obtain histological sections of Test and Control Artery samples. The resulting histological sections of Test and Control Artery samples were mounted on glass slides and cover-slipped using Fluoromount-G™ solution (17984-25, Electron Microscopy Sciences, Hatfield, PA).

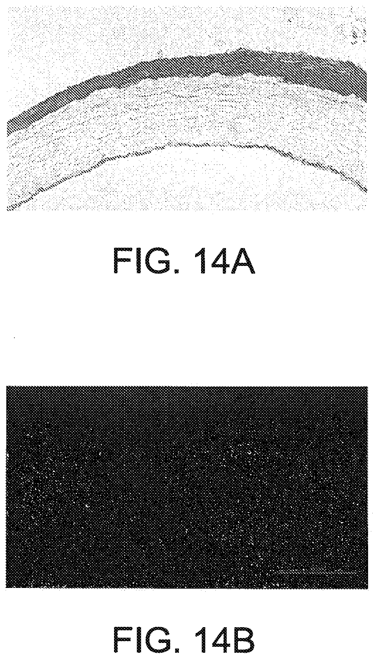

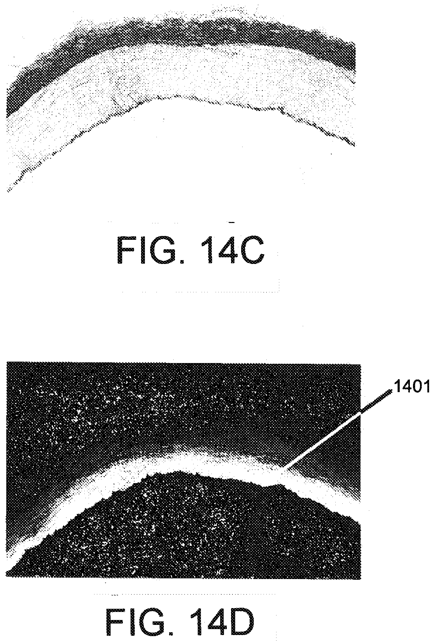

The histological sections of the Control and Test Artery are shown in Figures 14A and 14C, respectively. Fluorescence micrographs (596nm excitation, 615nm emission) of these images are shown in Figures 14B and 14D, respectively. The Test Artery section exhibited fluorescence (Figure 14D, as depicted by arrow 1401), due to transfer of the Texas-red-labeled-dextran to the artery during Device 12 inflation. The Control Artery section (Figure 14B) had no contact with Device 12 and exhibited no fluorescence, hence why this Figure is dark.

### Example 13: In Vivo Delivery of Texas-Red-Labeled-Dextran to a Canine Artery from a Coated Balloon Catheter

A structural cover (as prepared in Example 1) was assembled onto a balloon catheter (as described in Example 2). A hydrophilic coating was then applied to the balloon catheter as described in Example 11. As noted in Example 11, this coating contained a fluorescent molecule (Texas-red-labeled-dextran). An outer sheath layer (previously prepared per Example 4) was then assembled onto the coated balloon catheter (per Example 5). This balloon catheter is herein referred to as Device 13.

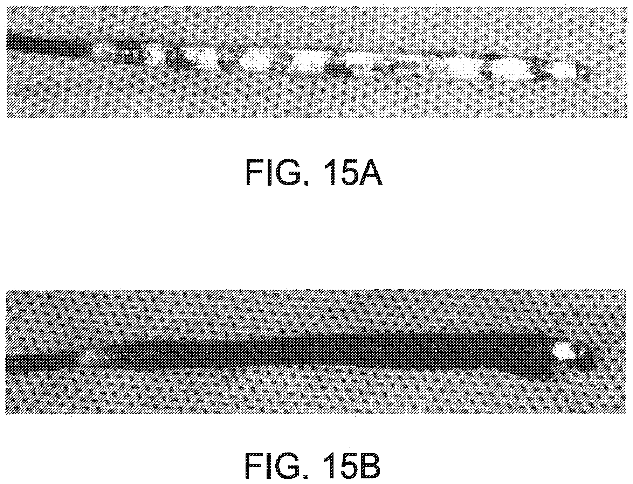

Device 13 was inserted into a canine aorta and allowed to dwell for 15 minutes without inflation. After this time, Device 13 was removed from the animal and photographed (Figure 15A). At this time, Device 13 was not completely wet.

Then Device 13 was inserted into the iliac artery. The balloon was inflated to 12 atm for 1 minute. Device 13 was then deflated, removed from the canine, and photographed (Figure 15B). At this time, Device 13 was black in color indicating complete wetting.

The animal remained in life for approximately 4 hours. After this time, the animal was euthanized. The ballooned section of iliac artery was harvested and placed in a buffered formalin solution (10% Neutral Buffered Formalin, VWR, Cat# BDH0502-20L, West Chester PA). This artery is herein defined as the Test Iliac Artery. An untreated section of iliac artery was harvested and placed in a buffered formalin solution (10% Neutral Buffered Formalin, VWR, Cat# BDH0502-20L, West Chester PA). This artery is herein defined as the Control Iliac Artery.

The Test and Control Iliac Arteries were separately sectioned. Light micrographs of the Test and Control Arteries are shown in Figures 16C and 16A, respectively. The histological sections were examined and photographed using fluorescence microscopy (596 nm excitation, 615 nm emission). The Test Iliac Artery section (Figure 16C) exhibited fluorescence (Figure 16D, as depicted by arrow 1601), due to transfer of the Texas-red-labeled-dextran to the artery during Device 13 inflation. The Control Iliac Artery section (Figure 16A) had no contact with Device 13 and exhibited no fluorescence (Figure 16B), hence why this Figure is dark.

### Example 14: In-Vitro Evaluation of Pre-Hydration of the PVA Coating Prior to First and Second Inflation

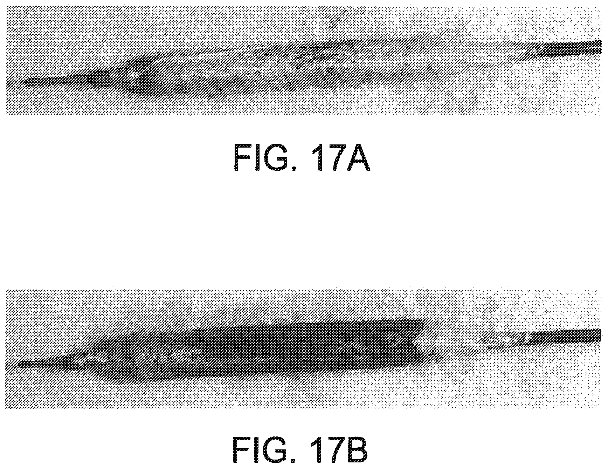

Device 14 (as depicted in Figure 17A and 17B) was built according to Example 5 and tested in a manner similar to Example 7, except that the Device was not presoaked in blood for 20 minutes in its first state in order to avoid any possibility of pre-hydration of the PVA coating proceeding first inflation. The results of this experiment are detailed in Table 2 and Figures 17A and 17B.

The testing began with inflation to 6 atm for 1 minute in blood in a rigid tube. After this time, the degree of wetting was noted, and a picture of Device 14 was taken (Figure 17A). A subsequent inflation to 12 atm for 1 minute in blood in a rigid tube followed. The degree of wetting was recorded and a picture (Figure 17B) was taken.

For comparison, Table 2 summarizes the degree of wetting of devices with and without prehydration (Devices 8a and 14, respectively). As noted above, prehydration of Device 8a (Example 8) was facilitated by incubating this device in blood for 20 minutes at its first state prior to device inflation. Device 14 was not incubated in blood at its first state prior to device inflation.

**Table 2 - Degree of Wetting With and Without Prehydration**

| | **Degree of Wetting After:** | | |
|---|---|---|---|
| | **20 Min Dwell** | **6 atm - 1 min** | **12 atm - 1 min** |
| **Device 14 No Prehydration** | **N/A** | **1 (****Figure 17A****)** | **5 (****Figure 17B****)** |
| **Device 8a With Prehydration** | **1** | **5 (****Figure 18A****)** | **10 (****Figure 18B****)** |

This example demonstrates that the outer sheath allows for a degree of coating hydration during the 20 minute dwell at first state and, that although this hydration does not cause excessive wet-out prematurely at first state (Table 2), it does allow for more rapid wetting during the first and second inflations to full diameter.

### Example 15: In Vivo Delivery of Texas-Red-Labeled-Dextran to a Femoral Artery from a Coated Balloon Catheter

A structural cover (as prepared in Example 1) was assembled onto a balloon catheter (as described in Example 2). A hydrophilic coating was then applied to the balloon catheter as described in Example 11. As noted in Example 11, this coating contained a fluorescent molecule (Texas-red-labeled-dextran). An outer sheath layer (previously prepared per Example 4) was then assembled onto the coated balloon catheter (per Example 5). This balloon catheter is herein referred to as Device 15.

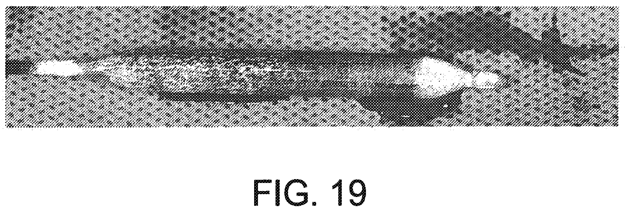

Device 15 was inserted into a canine femoral artery and immediately inflated to 6 atm for 1 minute. After this time, Device 15 was removed from the animal, rinsed with saline, re-inflated to 6 atm, and photographed (Figure 19). At this time, Device 15 was wet-out. As shown in Figure 19, Texas-red-labeled dextran could be seen on the outer most surface of the device, indicating that the coating became hydrated and was transferred through the outer sheath.

### Example 16: In Vitro Wetting of a Balloon Catheter Coated with a Thixotropic Gel

This Example describes the delivery of a thixotropic gel material to a vascular site from a coated balloon.

A first solution (referred herein as Solution 16A) was prepared by mixing phosphate buffered saline (PBS) (0.15M NaCl, pH 7.4, Invitrogen Corporation Carlsbad, CA) with 0.40 g/ml hydroxypropyl-β-cyclodextrin (HPβCD) (Sigma-Aldrich, St. Louis, MO) and 0.20 g/ml α-cydodextrin (a-CD) (Sigma-Aldrich) through stirring and heating (60° C).

A second solution (referred herein as Solution 16B) was prepared by dissolving polyethylene glycol (PEG, Dow Chemical, Midland, MI) of average Mn = 8kDa (0.26 g/ml) with PBS.

Equal volumes of Solution 16A and Solution 16 B were combined with mixing to form Gel Material A. Gel Material A was turbid, and was opaque and white in appearance
A structural cover was prepared as described in Example 1 and then assembled onto a balloon catheter as described in Example 2. This device (Device 16) was then coated with Gel Material A according to the method described below.

Device 16 was dipped into Gel Material A for about 10 seconds while rotating. After this time, the device was removed from Gel Material A. While rotating the device, a heat gun was used to blow warm air (about 40° C) over the device for approximately 3 minutes. This process was then repeated two additional times. Next, the device was allowed to air dry overnight.

An outer sheath was prepared as described in Example 4 and then assembled onto Device 16 as described in Example 5. Device 16 underwent testing for *in vitro* blood wetting according to the method described below.

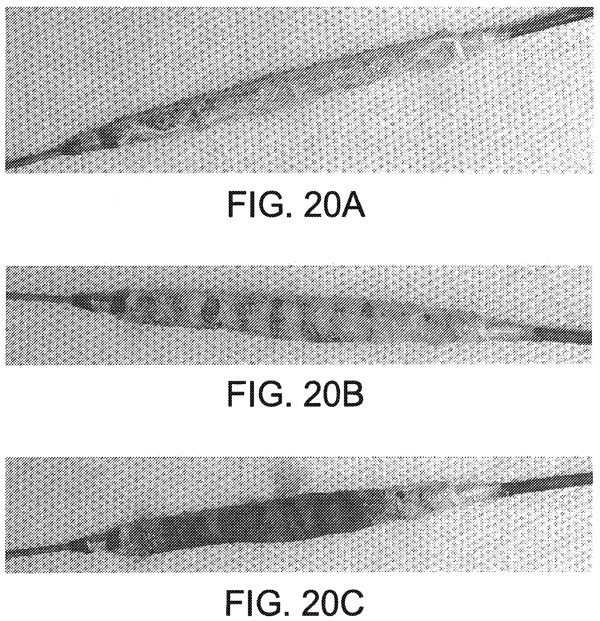

Blood was harvested from a canine, citrated to prevent clotting, and placed in a 50 ml vial. Device 16 was fully submerged in the blood at first state (unexpanded) for 20 minutes. After 20 minutes, Device 16 was removed from the blood, fully rinsed with saline, and photographed (Figure 20A).

Device 16 (which has a nominal inflation diameter of 6 mm) was inserted into a 5.9 mm diameter rigid tube (70 mm in length) in blood. Device 16 was then inflated to 6 atm for 1 minute. Afterward, Device 16 was deflated, removed from the blood, rinsed with saline, and photographed (Figure 20B). After rinsing, Device 16 was re-inserted into the 5.9 mm diameter rigid tube (70 mm in length) in blood and re-inflated to 12 atm for 1 minute. Afterward, Device 16 was deflated, removed from the blood, rinsed with saline, and photographed (Figure 20C). As shown in Figure 20C, Device 16 was fully wet at this time.

This example demonstrates that a thixotropic gel coating formulation enables wetting of the device of the agent-eluting invention.

### Example 17: Additional Formulations

The coating formulation detailed in Example 16 may be modified to include one or more therapeutic agents. It is expected devices of the agent-eluting invention, coated with these modified formulations will perform as that device described in Example 16 and deliver to target tissues an effective dose of the agent(s).

A first formulation (referred to herein as "Formulation 17A") is prepared by mixing phosphate buffered saline (PBS) (0.15M NaCl, pH 7.4, Invitrogen Corporation Carlsbad, CA) with 0.40 g/ml hydroxypropyl-β-cyclodextrin (HPβCD) (Sigma-Aldrich, St. Louis, MO) and 0.20 g/ml α-cydodextrin (α-CD) (Sigma-Aldrich) through stirring and heating (60° C), followed by adding dexamethasone (Pharmacia & Upjohn Company, Kalamazoo, MI) at 20 mg/ml with stirring and heating (60° C).

A second formulation (referred herein as "Formulation 17B") is prepared by dissolving polyethylene glycol (PEG, Dow Chemical, Midland, MI) of average Mn = 8kDa (0.26 g/ml) with PBS.

Equal volumes of Formulation 17A and Formulation 17B are combined *via* mixing to form a gel, herein referred to as "Material B".

A structural cover is prepared as described in Example 1 and assembled onto a balloon catheter as described in Example 2. This device (hereinafter "Device 17") is then coated with Material B according to the method described in Example 16. Device 17 is tested for *in vitro* blood wetting according to the method described in Example 16.

This example may be repeated changing only the composition of the "first formulation" (detailed above) as follows.

An alternative first formulation may be prepared by mixing PBS (0.15M NaCl, pH 7.4, Invitrogen) with 0.40 g/ml hydroxypropyl-β-cyclodextrin (HPβCD) (Sigma-Aldrich, St. Louis, MO) and 0.20 g/ml α-cydodextrin (α-CD) (Sigma-Aldrich) through stirring and heating (60° C), followed by adding 17β-estradiol (20 mg/ml) (Sigma-Aldrich) and then stirring and heating (60° C).

Another alternative first formulation may be prepared by mixing PBS (0.15M NaCl, pH 7.4) with 0.40 g/ml hydroxypropyl-β-cyclodextrin (HPβCD) (Sigma-Aldrich, St. Louis, MO) and 0.20 g/ml α-cydodextrin (α-CD) (Sigma-Aldrich) through stirring and heating (60° C), followed by adding dicumarol (0.67 mg/ml) (Sigma-Aldrich) by stirring and heating (60° C).

An alternative first formulation may be prepared by mixing PBS (0.15M NaCl, pH 7.4) with 0.40 g/ml hydroxypropyl-β-cyclodextrin (HPβCD) (Sigma-Aldrich, St. Louis, MO) and 0.20 g/ml α-cydodextrin (α-CD) (Sigma-Aldrich) through stirring and heating (60° C), followed by adding rapamycin (0.40 mg/ml) (Sigma-Aldrich) by stirring and heating (60° C).

Another first formulation may be prepared by mixing PBS (0.15M NaCl, pH 7.4) with 0.40 g/ml hydroxypropyl-β-cyclodextrin (HPβCD) (Sigma-Aldrich, St. Louis, MO) and 0.20 g/ml α-cydodextrin (α-CD) (Sigma-Aldrich) through stirring and heating (60° C), followed by adding everolimus (0.20 mg/ml) (Sigma-Aldrich) and stirring and heating (60° C).

### Example 18: In vivo Drug Delivery

This example demonstrates *in vivo* drug delivery using several different drug eluting balloon catheters of the present invention.

Twelve drug eluting balloon catheters were constructed and deployed *in vivo* as described below.

Twelve structural covers were prepared as follows. For each structural cover, a film tube was made of an elastomer-imbibed ePTFE film as described in the commonly-assigned, co-pending U.S. Patent Publication 20080125710, entitled INFLATABLE IMBIBED POLYMER DEVICES. Seven layers of the film, 20 cm wide, were longitudinally wrapped on a 1.9 mm stainless steel mandrel with the machine direction of the film parallel to the longitudinal axis of the mandrel. This film tube was overwrapped with approximately 2 layers of a sacrificial ePTFE film to prevent the tube from wrinkling in the subsequent steps. The mandrel was heated in an oven set at 225° C for 1.75 minutes and the sacrificial ePTFE layers were then removed. Each structural cover construct was removed from the mandrel and cut to a 6.0 cm length.

A dexamethasone coating formulation containing 0.40 g/g deionized water, 0.56 g/g hydroxypropyl-β-cyclodextrin (HPβCD, Sigma-Aldrich, St. Louis, MO), and 0.03 g/g dexamethasone (Pharmacia & UpJohn Co, Bridgewater, New Jersey), was prepared by placing appropriate quantities of each component in a beaker and stirring overnight at room temperature. This coating formulation is herein referred to as Formulation Dex-ACD.

A paclitaxel coating formulation containing 0.62 g/g deionized water, 0.37 g/g hydroxypropyl-β-cyclodextrin (HPβCP, Sigma-Aldrich, St. Louis, MO), and 1.41 mg/g paclitaxel (LC Laboratories, Woburn, MA), was prepared by placing appropriate quantities of each component in a beaker and stirring overnight at room temperature. This coating formulation is herein referred to as Formulation Ptx-ACD.

A paclitaxel coating formulation containing 0.73 g/g methanol, 0.22 g/g hydroxypropyl-β-cyclodextrin (HPβCD, Sigma-Aldrich, St. Louis, MO), and 58.58 mg/g paclitaxel (LC Laboratories, Woburn, MA), was prepared by placing appropriate quantities of each component in a beaker and stirring overnight at room temperature. This coating formulation is herein referred to as Formulation Ptx-MCD.

A paclitaxel coating formulation containing 0.75 g/g methanol, 0.19 g/g sodium salicylate (Sigma-Aldrich, St. Louis, MO), and 59.29 mg/g paclitaxel (LC Laboratories, Woburn, MA), was prepared by placing appropriate quantities of each component in a beaker and stirring overnight at room temperature. This coating formulation is herein referred to as Formulation Ptx-MNS.

Each structural cover (prepared as described above) was separately slipped over a mandrel which was subsequently rotated. While the covers were rotating, 100 µl of one of the formulation Dex-ACD, Ptx-ACD, Ptx-MCD, or Ptx-MNS was applied to a 40 mm length mid section of the structural cover according to the schedule shown in Table 3. Each coated cover was then dried in an oven at approximately 75° C for 20 minutes.

An ePTFE film was obtained having the following characteristics. Width (parallel to the machine direction): 10 cm. Matrix tensile strength, machine direction: 101,087 psi. Density: 0.415 g/cc. The typical estimated mean fibril length for this film material is 32 µm, arrived at by examination of a scanning electron photomicrograph of the material.

This ePTFE film was used to prepare twelve outer sheaths as follows. For each sheath, a film tube was created by longitudinally-wrapping two layers of the film characterized above onto a 2.5 mm diameter mandrel with the machine direction of the film parallel to the longitudinal axis of the mandrel. This film was overwrapped with approximately 1 layer of a sacrificial ePTFE. The film-covered mandrel was heated in an oven set at 380° C for 6 minutes and then the sacrificial ePTFE layer was removed. This sheath construct was removed from the mandrel and cut to a 6.0 cm length.

Each of the twelve outer sheaths was modified with a hydrophilic coating using the method essentially as described in co-assigned U.S. Patent 7,020,529, entitled "Defibrillation Electrode Cover". Sheaths were fully submerged in a bath of 100% isopropyl alcohol for 30 seconds, then transferred to a bath containing 2% polyvinyl alcohol (g/mL) in deionized water and allowed to dwell for 20 minutes. Sheaths were then rinsed in deionized water for 15 minutes. Upon rinse completion, the sheaths were transferred to a bath containing 2% glutaraldehyde (mL/mL) and 1.0% hydrochloric acid (mL/mL) in deionized water. The sheaths remained in this bath for 15 minutes and were then transferred to a deionized water rinse for an additional 15 minutes. All sheaths were allowed to dry in ambient air for approximately 2 hours

Twelve balloon catheters (Bavaria Medizin Technologie, Oberpfaffenhofen, Germany, model # BMT-035, with balloon dimensions of 6.0 mm x 40 mm) were obtained. One coated structural cover (see Table 3, below) was centered over each balloon aligning the distal and proximal ends of the drug coating with the balloon marker bands. Loctite 7701 Primer (Henkel AG & Co. KgaA, Düsseldorf, Germany) was applied to the end of the coated structural layer and surrounding catheter. The ends of the coated structural layer were then fixedly attached to the balloon catheter using approximately five layers of an approximately 6.4 mm width of ePTFE reinforcing film. The reinforcing film layers were wrapped circumferentially around the cover ends while Loctite 4981 was applied to the film.

One outer sheath was then placed over the coated structural cover (now attached to a balloon catheter) with their ends aligned. Loctite 7701 Primer (Henkel AG & Co. KgaA, Düsseldorf, Germany) was applied to the end of the outer sheath and surrounding catheter. The ends of the outer sheath were then fixedly attached to the balloon catheter using approximately five layers of an approximately 6.4 mm width of ePTFE reinforcing film. The reinforcing film layers were wrapped circumferentially around the outer sheath ends while Loctite 4981 was applied to the film.

Each balloon catheter was deployed in a porcine femoral artery as described below.

Prior to surgery, angiography of each treatment site was used to obtain vessel diameter and length measurements and to determine the appropriate balloon inflation pressure required for approximately 20-30% oversizing. Each balloon catheter was tracked to the treatment site and inflated for 60 seconds, and subsequently, deflated and removed from the animal. The animal remained in life under anesthesia for at least 1 hour with blood flow through the treatment site.

After this time period, each animal was euthanized. Then, the treated arterial vessel segment was exposed, attached to a longitudinal retention device, and excised. An untreated, remote artery (the carotid artery) was also harvested to assess potential systemic drug delivery to a remote site.

Adipose tissue was removed from the adventitia of each harvested arterial segment. Then, radial cross-sections (100 ± 50 mg) were carefully cut from each treated and control artery. The mass of each section and its location along the treatment length were noted. Vessel sections distal and proximal to the treatment areas were also harvested.

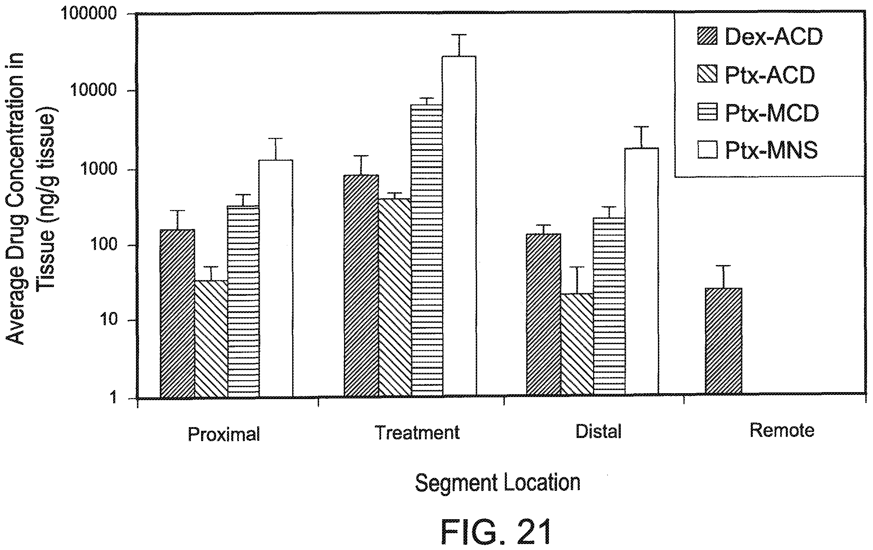

Arteries treated with devices containing paclitaxel (see Table 3) were analyzed for paclitaxel concentration by LC/MS-MS. Arteries treated with devices containing dexamethasone were analyzed for dexamethasone concentration by LC/MS-MS. For each treated artery, mean drug concentrations in the proximal, treated, distal, and remote segments were calculated as the average drug concentration of all sections in the indicated segment (Table 3). Treatment means (Figure 21) were then calculated by averaging the segment means with n=3 arteries for each treatment group.

**Table 3: Summary of Drug Concentrations (ng drug per g tissue) in Arterial Segments Proximal to, Within the Treatment Site, Distal to, or Remote from Tissue Treated by Balloon Catheter Deployment**

| Avg Dexamethasone Per Segment (ng drug/g tissue) | | | | | | |
|---|---|---|---|---|---|---|
| Structural Cover/Device ID | Coating Formulation on Balloon Catheters | Artery | Proximal | Treatment | Distal | Remote |
| 1498-166-19 | Formulation Dex-ACD | 1 | 69 | 280 | 131 | 0 |
| 1498-166-25 | | 2 | 81 | 1408 | 168 | 23 |
| 1498-166-26 | | 3 | 322 | 711 | 94 | 49 |

| Avg Paclitaxel Per Segment (ng drug/g tissue) | | | | | | |
|---|---|---|---|---|---|---|
| Structural Cover/Device ID | Coating Formulation on Balloon Catheters | Artery | Proximal | Treatment | Distal | Remote |
| DEB356 | Formulation Ptx-ACD | 4 | 48 | 355 | 0 | 0 |
| DEB358 | | 5 | 37 | 327 | 49 | 0 |
| DEB353 | | 6 | 13 | 456 | 15 | 0 |
| DEB502 | Formulation Ptx-MCD | 7 | 178 | 4905 | 273 | 0 |
| DEB506 | | 8 | 325 | 5800 | 107 | 0 |
| DEB504 | | 9 | 451 | 8080 | 227 | 0 |
| DEB496 | Formulation Ptx-MNS | 10 | 2256 | 48750 | 3121 | 0 |
| DEB494 | | 11 | 286 | 3680 | 211 | 0 |
| DEB495 | | 12 | 1446 | 31750 | 1968 | 0 |

As shown in Table 3 and Figure 21, deployment of each balloon catheter successfully delivered drug to the treatment site with minimal drug delivery to adjacent (proximal or distal) or remote vascular tissue sites.

### Example 19: Alternative Formulations

This example depicts *in vivo* drug delivery using drug eluting balloon catheters of the present invention which use therapeutic agent formulations different from those in Example 18.

Drug eluting balloon catheters are constructed and deployed *in vivo* as described in Example 18, above. However the following drug formulations may be substituted for those described in Example 18.

A 17β-estradiol coating formulation containing 0.62 g/g DI water, 0.37 g/g hydroxypropyl-β-cyclodextrin (HPβCD, Sigma-Aldrich, St. Louis, MO), and 1.41 mg/g 17β-estradiol (Sigma-Aldrich, St. Louis, MO) is prepared by placing appropriate quantities of each component in a beaker and stirring overnight at room temperature.

A 17β-estradiol coating formulation containing 0.73 g/g methanol, 0.22 g/g hydroxypropyl-β-cyclodextrin (HPβCD, Sigma-Aldrich, St. Louis, MO), and 50.0 mg/g 17β-estradiol (Sigma-Aldrich, St. Louis, MO) is prepared by placing appropriate quantities of each component in a beaker and stirring overnight at room temperature.

A 17β-estradiol coating formulation containing 0.75 g/g methanol, 0.19 g/g sodium salicylate (Sigma-Aldrich, St. Louis, MO), and 50.0 mg/g 17β-estradiol (Sigma-Aldrich, St. Louis, MO) is prepared by placing appropriate quantities of each component in a beaker and stirring overnight at room temperature.

A dicumarol coating formulation containing 0.62 g/g DI water, 0.37 g/g hydroxypropyl-β-cyclodextrin (HPβCD, Sigma-Aldrich, St. Louis, MO), and 0.40 mg/g dicumarol (Sigma-Aldrich, St. Louis, MO) is prepared by placing appropriate quantities of each component in a beaker and stirring overnight at room temperature.

A dicumarol coating formulation containing 0.73 g/g methanol, 0.22 g/g hydroxypropyl-β-cyclodextrin (HPβCD, Sigma-Aldrich, St. Louis, MO), and 0.40 mg/g dicumarol (Sigma-Aldrich, St. Louis, MO) is prepared by placing appropriate quantities of each component in a beaker and stirring overnight at room temperature.

A dicumarol coating formulation containing 0.75 g/g methanol, 0.19 g/g sodium salicylate (Sigma-Aldrich, St. Louis, MO), and 0.40 mg/g dicumarol (Sigma-Aldrich, St. Louis, MO) is prepared by placing appropriate quantities of each component in a beaker and stirring overnight at room temperature.

A rapamycin coating formulation containing 0.62 g/g DI water, 0.37 g/g hydroxypropyl-β-cyclodextrin (HPβCD, Sigma-Aldrich, St. Louis, MO), and 0.40 mg/g rapamycin (Sigma-Aldrich, St. Louis, MO) is prepared by placing appropriate quantities of each component in a beaker and stirring overnight at room temperature.

A rapamycin coating formulation containing 0.73 g/g methanol, 0.22 g/g hydroxypropyl-β-cyclodextrin (HPβCD, Sigma-Aldrich, St. Louis, MO), and 0.40 mg/g rapamycin (Sigma-Aldrich, St. Louis, MO) is prepared by placing appropriate quantities of each component in a beaker and stirring overnight at room temperature.

A rapamycin coating formulation containing 0.75 g/g methanol, 0.19 g/g sodium salicylate (Sigma-Aldrich, St. Louis, MO), and 0.40 mg/g rapamycin (Sigma-Aldrich, St. Louis, MO) is prepared by placing appropriate quantities of each component in a beaker and stirring overnight at room temperature.

A everolimus coating formulation containing 0.62 g/g DI water, 0.37 g/g hydroxypropyl-β-cyclodextrin (HPβCD, Sigma-Aldrich, St. Louis, MO), and 0.20 mg/g everolimus (Sigma-Aldrich, St. Louis, MO) is prepared by placing appropriate quantities of each component in a beaker and stirring overnight at room temperature.

A everolimus coating formulation containing 0.73 g/g methanol, 0.22 g/g hydroxypropyl-β-cyclodextrin (HPβCD, Sigma-Aldrich, St. Louis, MO), and 0.20 mg/g everolimus (Sigma-Aldrich, St. Louis, MO) is prepared by placing appropriate quantities of each component in a beaker and stirring overnight at room temperature.

A everolimus coating formulation containing 0.75 g/g methanol, 0.19 g/g sodium salicylate (Sigma-Aldrich, St. Louis, MO), and 0.20 mg/g everolimus (Sigma-Aldrich, St. Louis, MO) is prepared by placing appropriate quantities of each component in a beaker and stirring overnight at room temperature.

### Example 20: Microstructural Changes

The following example shows the microstructural changes which occur upon expansion of drug eluting balloons of the present invention.

A drug eluting balloon was prepared as described in Example 18 but the structural cover was not coated with a formulation containing a therapeutic agent. Figure 3C is a scanning electromicrograph (at magnification of 500x) of the film comprising the outer sheath mounted on this balloon as assembled and prior to inflation. It will be noted the microstructure is in a first state with a closed microstructure. The balloon was subsequently expanded to its nominal diameter (6.0 mm) and the scanning electromicrograph of the film comprising the outer sheath at said expanded state is shown in Figure 3D. As is apparent, a second state results from expansion, *i.e.,* a film with a more open microstructure.

### Example 21: In Vivo Drug Delivery from Various Paclitaxel Coating Formulations

Fourteen drug eluting balloon catheters were constructed and deployed *in vivo* as described below.

Eight structural covers were prepared as follows (*see* Table 5 for structural cover IDs). For each structural cover, a film tube was made of an elastomer-imbibed ePTFE film as described in the commonly-assigned, co-pending U.S. Patent Publication 20080125710, entitled INFLATABLE IMBIBED POLYMER DEVICES. Seven layers of the film, 20 cm wide, were longitudinally wrapped on a 1.9 mm stainless steel mandrel with the machine direction of the film parallel to the longitudinal axis of the mandrel. This film tube was overwrapped with approximately 2 layers of a sacrificial ePTFE film to prevent the tube from wrinkling in the subsequent steps. The mandrel was baked in an oven set at 225° C for 1.75 minutes and the sacrificial ePTFE layers were then removed. Each structural cover construct was removed from the mandrel and cut to a 6.0 cm length.

Six structural covers were prepared as follows (*see* Table 5 for structural cover IDs). For each structural cover, a film tube was made of an elastomer-imbibed ePTFE film as described in the commonly-assigned, co-pending U.S. Patent Publication 200801257, entitled INFLATABLE IMBIBED POLYMER DEVICES. Five layers of the film, 20 cm wide, were longitudinally wrapped on a 1.7 mm stainless steel mandrel with the machine direction of the film parallel to the longitudinal axis of the mandrel. This film tube was overwrapped with approximately 2 layers of a sacrificial ePTFE film to prevent the tube from wrinkling in the subsequent steps. The mandrel was baked in an oven set at 225° C for 1.75 minutes and the sacrificial ePTFE layers were then removed. Each structural cover construct was removed from the mandrel and cut to a 6.0 cm length.

The following paclitaxel coating formulations were prepared and are summarized in Table 4.

A paclitaxel coating formulation containing 0.72 g/g methanol, 0.21 g/g hydroxypropyl-β-cyclodextrin (HPβCD, Sigma-Aldrich, St. Louis, MO), 0.01 g/g dimethyl sulfoxide (DMSO, Sigma-Aldrich, St. Louis, MO), and 58.6 mg/g paclitaxel (LC Laboratories, Woburn, MA) was prepared by placing appropriate quantities of each component in a beaker and stirring until dissolved. This coating formulation is herein defined as **Formulation Ptx-MCD+.**

A paclitaxel coating formulation containing 0.70 g/g methanol, 0.19 g/g sodium salicylate (NS, Sigma-Aldrich, St. Louis, MO), 0.36 g/g DMSO, and 69.4 mg/g paclitaxel was prepared by placing appropriate quantities of each component in a beaker and stirring until dissolved. This coating formulation is herein defined as Formulation Ptx-MNS4+.

A paclitaxel coating formulation containing 0.74 g/g ethanol (EMD, Rockland, Ma), 0.07 g/g water, 20.0 mg/g paclitaxel, 0.07 g/g HPβCD, 3.2 mg/g DMSO, and 0.10 g/g poloxamer-188 (Lutrol F68, Mutchler Inc, Harrington Park, NJ) was prepared by placing appropriate quantities of each component in a beaker and stirring until dissolved. This coating formulation is herein defined as Formulation Ptx- Pol/CD/DMSO-30.

A paclitaxel coating formulation containing 0.72 g/g ethanol (EMD, Rockland, Ma), 0.04 g/g water, 30.5 mg/g paclitaxel, 0.05 g/g HPβCD, 18.9 mg/g DMSO, and 0.14 g/g poloxamer-188 was prepared by placing appropriate quantities of each component in a beaker and stirring until dissolved. This coating formulation is herein defined as Formulation Ptx- Pol/CD/DMSO-40.

A paclitaxel coating formulation containing 0.76 g/g methanol, 39.6 mg/g paclitaxel, 0.20 g/g HYAMINE®-1622 (Product# 53751, Sigma-Aldrich, St. Louis, MO) was prepared by placing appropriate quantities of each component in a beaker and stirring until dissolved. This coating formulation is herein defined as Formulation Ptx- HYA.

A paclitaxel coating formulation containing 0.87 g/g methanol, 43.5 mg/g paclitaxel, 0.08 g/g poloxamer-188, and 0.02 g/g polyethylene glycol (PEG, M_{w}=3350 Da, Product# 166978, The Dow Chemical Company, Pittsburg, CA) was prepared by placing appropriate quantities of each component in a beaker and stirring until dissolved. This coating formulation is herein defined as Formulation Ptx-PoPEG.

Upon completion of stirring, all coating formulations were clear solutions without any visible precipitation.

**Table 4: Paclitaxel Coating Formulations Examined in Example 21**

| **Formulation** | **Paclitaxel Coating Formulations (g component per g total)** | | | | | | | | | |
|---|---|---|---|---|---|---|---|---|---|---|
| | **Methanol** | **Ethanol** | **Water** | **Paclitaxel** | **HPβCD** | **NS** | **DMSO** | **Poloxamer** | **Hyamine** | **PEG** |
| **Ptx-MCD+** | 0.7214 | - | - | 0.0586 | 0.2100 | - | 0.0100 | - | - | - |
| **Ptx-MNS4+** | 0.7030 | - | - | 0.0694 | - | 0.1918 | 0.0359 | - | - | - |
| **Ptx- Pol/CD/DMSO-30** | - | 0.7428 | 0.0687 | 0.0200 | 0.0659 | - | 0.0032 | 0.0995 | - | - |
| **Ptx- Pol/CD/DMSO-40** | - | 0.7173 | 0.0442 | 0.0305 | 0.0490 | - | 0.0189 | 0.1400 | - | - |
| **Ptx- HYA** | 0.7610 | - | - | 0.0396 | - | - | - | - | 0.1994 | - |
| **Ptx- PoPEG** | 0.8716 | - | - | 0.0435 | - | - | - | 0.0849 | - | 0.0241 |

Each structural cover (prepared as described above) was separately slipped over a mandrel which was subsequently rotated. While the covers were rotating, 100 µl of one of the paclitaxel formulations described above (and in Table 4) was applied to a 40 mm length mid-section of the structural cover according to the schedule shown in Table 5. Each coated cover was then dried in an oven at approximately 75° C for 20 minutes.

An ePTFE film tape was obtained having the following characteristics. Width (parallel to the machine direction): 10 cm. Matrix tensile strength, machine direction: 92,000 psi. Matrix tensile strength, transverse direction: 570 psi. Density: 0.52 g/cc. Mean fibril length: 30 µm, arrived at by examination of a scanning electron photomicrograph of the material.

This ePTFE film tape was used to prepare fourteen outer sheaths as follows. For each sheath, a film tube was created by longitudinally wrapping two layers of the film tape characterized above onto a 2.5 mm diameter mandrel with the machine direction of the film parallel to the longitudinal axis of the mandrel. This film was overwrapped with approximately 1 layer of a sacrificial ePTFE. The film-covered mandrel was baked in an oven set at 380° C for 6 minutes and then the sacrificial ePTFE layer was removed. This sheath construct was removed from the mandrel and cut to a 6.0 cm length.

Each of the fourteen outer sheaths was modified with a hydrophilic coating using the following method. Sheaths were fully submerged in a bath of 100% isopropyl alcohol for 30 seconds, then transferred to a bath containing 2% polyvinyl alcohol (g/mL) in deionized (DI) water and allowed to dwell for 20 minutes. Sheaths were then rinsed in DI water for 15 minutes. Upon rinse completion, the sheaths were transferred to a bath containing 2% glutaraldehyde (mL/mL) and 1% hydrochloric acid (mL/mL) in DI water. The sheaths remained in this bath for 15 minutes and were then transferred to a DI water rinse for an additional 15 minutes. All sheaths were allowed to dry in ambient air for approximately 2 hours.

Fourteen balloon catheters were obtained from either Bavaria Medizin Technologie (BMT, Oberpfaffenhofen, Germany, model # BMT-035, with balloon dimensions of 6.0 mm x 40 mm or 5.0 mm x 40 mm) or Creagh Medical, LTD (Galway, Ireland, model # PN00084-540L, with balloon dimensions of 5.0 mm x 40 mm) (see Table 5).

One coated structural cover (from Table 5) was centered over each balloon aligning the distal and proximal ends of the drug coating with the balloon marker bands. Loctite 7701 Primer (Henkel AG & Co. KgaA, Düsseldorf, Germany) was applied to the end of the coated structural layer and surrounding catheter. The ends of the coated structural layer were then fixedly attached to the balloon catheter using approximately five layers of an approximately 6.4 mm width of ePTFE reinforcing film. The reinforcing film layers were wrapped circumferentially around the cover ends while Loctite 4981 was applied to the film.

One outer sheath was then placed over the coated structural cover (now attached to a balloon catheter) with their ends aligned. Loctite 7701 Primer (Henkel AG & Co. KgaA, Düsseldorf, Germany) was applied to the end of the outer sheath and surrounding catheter. The ends of the outer sheath were then fixedly attached to the balloon catheter using approximately five layers of an approximately 6.4 mm width of ePTFE reinforcing film. The reinforcing film layers were wrapped circumferentially around the outer sheath ends while Loctite 4981 was applied to the film.

Each balloon catheter was deployed in a porcine femoral artery as described below.

Prior to surgery, angiography of each treatment site was used to obtain vessel diameter and length measurements and to determine the appropriate balloon inflation pressure required for approximately 20-30% oversizing. Each balloon catheter was tracked to the treatment site and inflated for 60 seconds, and subsequently, deflated and removed from the animal. The animal remained in life for either 1 hour or 24 hour with blood flow through the treatment site.

After this time period, each animal was euthanized. Then, the treated arterial vessel segment was exposed, attached to a longitudinal retention device, and excised. An untreated, remote artery (the carotid artery) was also harvested to assess potential systemic drug delivery to a remote site.

Adipose tissue was removed from the adventitia of each harvested arterial segment. Then, radial cross-sections (100 ± 50 mg) were carefully cut from each treated and control artery. The mass of each section and its location along the treatment length were noted. Vessel sections distal and proximal to the treatment areas were also harvested.

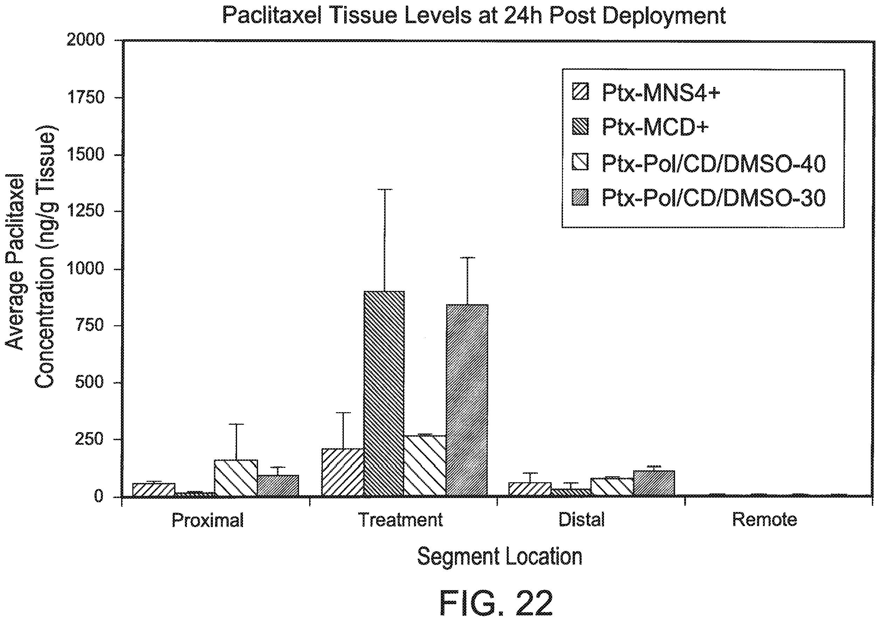

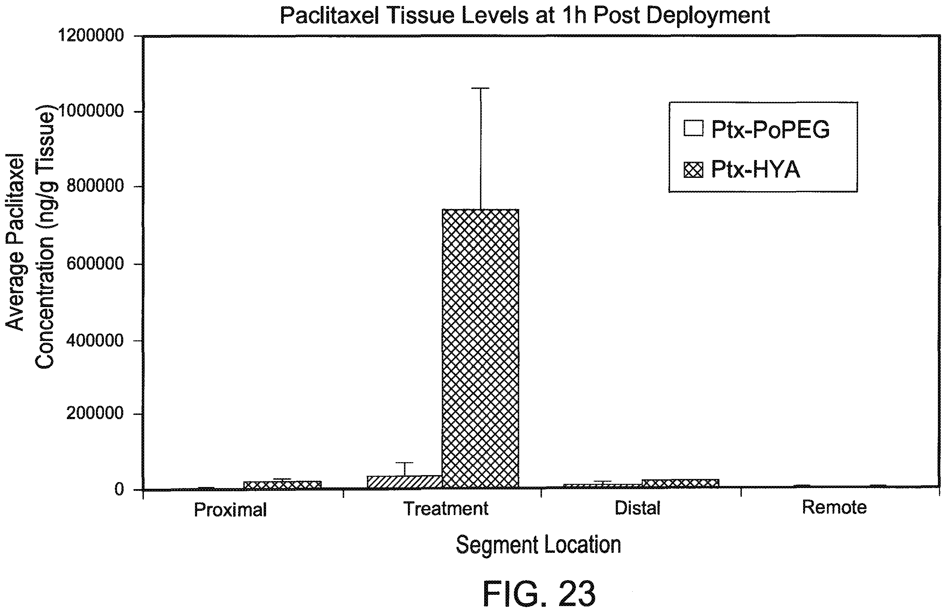

The vessel sections were analyzed for paclitaxel concentration by LC/MS-MS. For each treated artery, mean drug concentrations in the proximal, treated, distal, and remote segments were calculated as the average drug concentration of all sections in the indicated segment (Table 5). Treatment means were then calculated by averaging the segment means with n=2 arteries for each 24h treatment group (Figure 22) and n=3 arteries for each 1h treatment group (Figure 23).

As shown in Table 5 and Figures 22 and 23, deployment of each balloon catheter successfully delivered paclitaxel to the treatment site with minimal drug delivery to adjacent or remote vascular tissue sites.

**Table 5: Summary of Paclitaxel Concentrations (ng drug per g tissue) in Arterial Segments Proximal to, Within the Treatment Site, Distal to, or Remote from Tissue Treated by Balloon Catheter Deployment at 1h or 24h Post-Deployment**

| **Structural Cover/Device ID** | | **Coating Formulation** | **Balloon Manufacturer, Diameter** | **Time (Post Deployment)** | **Artery** | **Average Pacliaxel Per Segment (ng drug/g tissue)** | | | |
|---|---|---|---|---|---|---|---|---|---|
| **ID** | **# film layers, inner diameter** | | | | | **Proximal** | **Treatment** | **Distal** | **Remote** |
| DEB585 | 7 layers, 1.9 mm diameter (uninflated) | **Ptx-MNS4+** | BMT, 5 mm | 24h | 1203-L | 41 | 97 | 83 | 0 |
| DEB588 | | | | | 1204-R | 61 | 320 | 18 | 0 |
| DEB532 | | **Ptx-MCD+** | BMT, 6mm | | 1201-R | 9 | 581 | 18 | 0 |
| DEB531 | | | | | 1201-L | 14 | 1218 | 44 | 0 |
| DEB597 | | **Ptx- Pol/CD/DMSO-40** | BMT, 5 mm | | 1203-R | 269 | 264 | 81 | 0 |
| DEB601 | | | | | 1204-L | 33 | 254 | 63 | 0 |
| DEB529 | | **Ptx- Pol/CD/DMSO-30** | | | 1219-R | 111 | 695 | 120 | 0 |
| DEB530 | | | | | 1219-L | 65 | 988 | 92 | 0 |
| DEB746 | 5 layers, 1.7 mm diameter (uninflated) | **Ptx- PoPEG** | Creagh, 5mm | 1h | 1239-R | 482 | 75790 | 15746 | 0 |
| DEB747 | | | | | 1246-L | 2424 | 5120 | 567 | 0 |
| DEB745 | | | | | 1241-R | 340 | 11500 | 705 | 0 |
| DEB736 | | **Ptx- HYA** | | | 1239-L | 12726 | 949000 | 14367 | 0 |
| DEB738 | | | | | 1246-R | 24830 | 891500 | 16300 | 0 |
| DEB737 | | | | | 1241-L | 5538 | 351500 | 12800 | 0 |

### Example 22: Paclitaxel Coating Formulations

The following paclitaxel coating formulations were prepared (and are summarized in Table 6) as described below.

A paclitaxel coating formulation containing 0.87 g/g methanol, 44.4 mg/g paclitaxel, 0.09 g/g poloxamer-188 was prepared by placing appropriate quantities of each component in a beaker and stirring until dissolved. This coating formulation is herein defined as Formulation Ptx- POLO.

A paclitaxel coating formulation containing 0.86 g/g methanol, 41.7 mg/g paclitaxel, and 0.10 g/g Vitamin B3 (Niacinamide, USP Grade, Spectrum Chemicals & Laboratory Products, New Brunswick, NJ) was prepared by placing appropriate quantities of each component in a beaker and stirring until dissolved. This coating formulation is herein defined as Formulation Ptx- VB.

A paclitaxel coating formulation containing 0.82 g/g methanol, 39.5 mg/g paclitaxel, 0.04 g/g Vitamin E (α-Tocopherol, Product# T3251, Sigma-Aldrich, St. Louis, MO), and 0.10 g/g Vitamin B3, was prepared by placing appropriate quantities of each component in a beaker and stirring until dissolved. This coating formulation is herein defined as Formulation Ptx- VBE.

Upon completion of stirring, all coating formulations were clear solutions without any visible precipitation.

**Table 6: Paclitaxel Coating Formulations Examined in Example 22**

| **Formulation** | **Paclitaxel Coating Formulations (g component per g total)** | | | | |
|---|---|---|---|---|---|
| | **Methanol** | **Paclitaxel** | **Vitamin E** | **Vitamin B3** | **Poloxamer** |
| Ptx- POLO | 0.8676 | 0.0444 | - | - | 0.0880 |
| Ptx-VBE | 0.8204 | 0.0395 | 0.0399 | 0.1002 | - |
| Ptx-VB | 0.8550 | 0.0417 | - | 0.1033 | - |

Structural covers were prepared per the methods described in Example 21 and as detailed in Table 7. Each structural cover was separately slipped over a mandrel which was subsequently rotated. While the covers were rotating, 100 µl of one of the paclitaxel formulation described in Table 6 was applied to a 40 mm length mid-section of the structural cover. Each coated cover was then dried in an oven set at 75° C for 20 minutes.

Following the methods of Example 21, each coated structural cover was used in the construction of a drug elution balloon. In brief, outer sheaths were prepared as described in Example 21. Each outer sheath was modified with a hydrophilic coating using the methods described in Example 21.

Balloon catheters were obtained from either Bavaria Medizin Technologie (BMT, Oberpfaffenhofen, Germany, model # BMT-035, with balloon dimensions of 6.0 mm x 40 mm or 5.0 mm x 40 mm) or Creagh Medical, LTD (Galway, Ireland, model # PN00084-540L, with balloon dimensions of 5.0 mm x 40 mm).

One coated structural cover was then attached to one balloon catheter using the methods described in Example 21. One outer sheath was then placed over the coated structural cover (now attached to a balloon catheter) with their ends aligned. The outer sheath was attached to the balloon catheter per the methods of Example 21.

**Table 7: Drug-Eluting Balloons Built Using Formulations Examined in Example 22**

| **Structural Cover/ Device ID** | | **Coating Formulation** | **Balloon Manufacturer, Diameter** |
|---|---|---|---|
| **ID** | **# film layers, inner diameter** | | |
| DEB629 | 7 layers, 1.9 mm diameter (uninflated) | **Ptx-VB** | BMT, 5 mm |
| DEB630 | | | |
| DEB631 | | | |
| DEB642 | | **Ptx-VBE** | BMT, 5 mm |
| DEB643 | | | |
| DEB641 | | | |
| DEB727 | 5 layers, 1.7 mm diameter (uninflated) | **Ptx- POLO** | Creagh, 5mm |
| DEB728 | | | |
| DEB729 | | | |

### Example 23: Alternative Formulations

The following drug formulations may be substituted for those described in Example 21.

A rapamycin coating formulation containing 0.76 g/g methanol, 39.6 mg/g rapamycin (Sigma-Aldrich, St. Louis, MO), 0.20 g/g HYAMINE®-1622 (Product# 53751, Sigma-Aldrich, St. Louis, MO) is prepared by placing appropriate quantities of each component in an airtight beaker and stirring overnight.

A rapamycin coating formulation containing 0.87 g/g methanol, 43.5 mg/g rapamycin (Sigma-Aldrich, St. Louis, MO), 0.08 g/g poloxamer-188, and 0.02 g/g polyethylene glycol (PEG, M_{w}=3350 Da, Product# 166978, The Dow Chemical Company, Pittsburg, CA) is prepared by placing appropriate quantities of each component in an airtight beaker and stirring overnight.

An everolimus coating formulation containing 0.76 g/g methanol, 39.6 mg/g everolimus (Sigma-Aldrich, St. Louis, MO), and 0.20 g/g HYAMINE®-1622 is prepared by placing appropriate quantities of each component in an airtight beaker and stirring overnight.

An everolimus coating formulation containing 0.87 g/g methanol, 43.5 mg/g everolimus (Sigma-Aldrich, St. Louis, MO), 0.08 g/g poloxamer-188, and 0.02 g/g polyethylene glycol (PEG, M_{w}=3350 Da, Product# 166978, The Dow Chemical Company, Pittsburg, CA) is prepared by placing appropriate quantities of each component in an airtight beaker and stirring overnight.

A dicumarol coating formulation containing 0.76 g/g methanol, 39.6 mg/g dicumarol (Sigma-Aldrich, St. Louis, MO), 0.20 g/g HYAMINE®-1622 (Product# 53751, Sigma-Aldrich, St. Louis, MO) is prepared by placing appropriate quantities of each component in an airtight beaker and stirring overnight.

A dicumarol coating formulation containing 0.87 g/g methanol, 43.5 mg/g dicumarol (Sigma-Aldrich, St. Louis, MO), 0.08 g/g poloxamer-188, and 0.02 g/g polyethylene glycol (PEG, M_{w}=3350 Da, Product# 166978, The Dow Chemical Company, Pittsburg, CA) is prepared by placing appropriate quantities of each component in an airtight beaker and stirring overnight.

A zotarolimus coating formulation containing 0.76 g/g methanol, 39.6 mg/g zotarolimus (LC Laboratories, Woburn, MA), 0.20 g/g HYAMINE®-1622 (Product# 53751, Sigma-Aldrich, St. Louis, MO) is prepared by placing appropriate quantities of each component in an airtight beaker and stirring overnight.

A zotarolimus coating formulation containing 0.87 g/g methanol, 43.5 mg/g zotarolimus (LC Laboratories, Woburn, MA), 0.08 g/g poloxamer-188, and 0.02 g/g polyethylene glycol (PEG, M_{w}=3350 Da, Product# 166978, The Dow Chemical Company, Pittsburg, CA) is prepared by placing appropriate quantities of each component in an airtight beaker and stirring overnight.

A docetaxel coating formulation containing 0.76 g/g methanol, 39.6 mg/g docetaxel (Sigma-Aldrich, St. Louis, MO), and 0.20 g/g Hyamine-1622 (Product# 53751, Sigma-Aldrich, St. Louis, MO) is prepared by placing appropriate quantities of each component in an airtight beaker and stirring overnight.

A docetaxel coating formulation containing 0.87 g/g methanol, 43.5 mg/g docetaxel (Sigma-Aldrich, St. Louis, MO), 0.08 g/g poloxamer-188, and 0.02 g/g polyethylene glycol (PEG, M_{w}=3350 Da, Product# 166978, The Dow Chemical Company, Pittsburg, CA) is prepared by placing appropriate quantities of each component in an airtight beaker and stirring overnight.

A docetaxel coating formulation containing 0.62 g/g DI water, 0.37 g/g hydroxypropyl-β-cyclodextrin (HPβCD, Sigma-Aldrich, St. Louis, MO), and 0.40 mg/g docetaxel (Sigma-Aldrich, St. Louis, MO) is prepared by placing appropriate quantities of each component in a beaker and stirring overnight at room temperature.

A docetaxel coating formulation containing 0.73 g/g methanol, 0.22 g/g hydroxypropyl-β-cyclodextrin (HPβCD, Sigma-Aldrich, St. Louis, MO), and 0.40 mg/g docetaxel (Sigma-Aldrich, St. Louis, MO) is prepared by placing appropriate quantities of each component in a beaker and stirring overnight at room temperature.

## Claims

1. A balloon catheter comprising:
a. a balloon comprising a coating and a therapeutic agent on an outer surface of said balloon;
b. a sheath disposed around said balloon wherein said sheath has a microstructure composed of nodes interconnected by fibrils and wherein said sheath possesses a variably permeable microstructure, wherein in an unexpanded state, the sheath substantially limits transfer of said therapeutic agent;
c. wherein said coating and therapeutic agent are disposed between the surface of the balloon and the sheath; and
d. wherein when said sheath is expanded by the balloon, said sheath allows transfer of said coating and therapeutic agent through the permeable microstructure of the sheath.

2. The balloon catheter of claim 1, wherein said coating and therapeutic agent are transferred to an area external to said sheath.

3. The balloon catheter of claim 2, wherein said coating and therapeutic agent are transferred through said sheath and onto a target tissue, optionally wherein said coating remains substantially adhered to the target tissue after balloon deflation.

4. The balloon catheter of claim 1, wherein said sheath is treated with a wetting agent.

5. The balloon catheter of claim 4, wherein said wetting agent is polyvinyl alcohol or heparin, or wherein said sheath wets out before expansion but substantially limits transfer of said therapeutic agent external said sheath in the unexpanded state.

6. The balloon catheter of claim 1, wherein said sheath undergoes microscopic wetting in a vessel while said balloon and sheath are in the unexpanded state and being delivered to a desired location within a vessel.

7. The balloon catheter of claim 1, wherein bodily fluids substantially wet-out the sheath when said sheath is expanded.

8. The balloon catheter of claim 7, wherein said coating also wets the sheath when said sheath is expanded, or wherein substantially all of said sheath is wet by the time said sheath is fully expanded, or wherein said wetting of the sheath is facilitated when said sheath is in contact to a vessel wall.

9. The balloon catheter of claim 1, wherein said sheath comprises a fluoropolymer, optionally wherein the fluoropolymer is ePTFE.

10. The balloon catheter of claim 1, wherein said nodes are aligned longitudinally to the longitudinal axis of said balloon catheter and said fibrils are aligned circumferentially to said axis, optionally wherein the distance between said nodes increases as said sheath expands.

11. The balloon catheter of claim 1, wherein said nodes are aligned circumferentially to the longitudinal axis of said balloon catheter and said fibrils are aligned longitudinally to said axis; optionally
wherein the distance between said fibrils increases as the outer sheath expands, or said fibrils re-orient as said outer sheath expands.

12. The balloon catheter of claim 1, wherein said coating comprises a hydrophilic component.

13. The balloon catheter of claim 12, wherein said hydrophilic component raises the solubility point of a hydrophobic therapeutic agent, or wherein said coating comprises at least one component selected from the following group benzethonium chloride, PEG, poloxamer, sodium salicylate, and hydroxypropyl-β-cyclodextrin.

14. The balloon catheter of claim 1, wherein said therapeutic agent is a hydrophilic agent, or wherein said therapeutic agent is a hydrophobic agent; optionally wherein said hydrophobic agent is selected from the group consisting of taxane domain-binding drugs, such as paclitaxel, and rapamycin.

15. The balloon catheter of claim 1, wherein said balloon further comprises a structural layer, optionally wherein said structural layer comprises said coating and therapeutic agent; or wherein the microstructure of the sheath changes as said balloon expands

## Patentansprüche

1. Ballonkatheter, umfassend:
a. einen Ballon, umfassend eine Beschichtung und ein therapeutisches Mittel auf einer äußeren Oberfläche des Ballons;
b. eine um den Ballon angeordnete Hülse, wobei die Hülse eine Mikrostruktur aufweist, die aus durch Fibrillen verbundene Knoten besteht, und wobei die Hülse eine variabel durchlässige Mikrostruktur besitzt, wobei in einem nicht ausgedehnten Zustand die Hülse im Wesentlichen den Transfer des therapeutischen Mittels beschränkt;
c. wobei die Beschichtung und das therapeutische Mittel zwischen der Oberfläche des Ballons und der Hülse angeordnet sind; und
d. wobei, wenn die Hülse durch den Ballon ausgedehnt wird, die Hülse den Transfer der Beschichtung und des therapeutischen Mittels durch die durchlässige Mikrostruktur der Hülse zulässt.

2. Ballonkatheter nach Anspruch 1, wobei die Beschichtung und das therapeutische Mittel auf eine zur Hülse externe Fläche übertragen werden.

3. Ballonkatheter nach Anspruch 2, wobei die Beschichtung und das therapeutische Mittel durch die Hülse und auf ein Zielgewebe übertragen werden, optional wobei die Beschichtung nach dem Entleeren des Ballons im Wesentlichen weiter am Zielgewebe haftet.

4. Ballonkatheter nach Anspruch 1, wobei die Hülse mit einem Benetzungsmittel behandelt wird.

5. Ballonkatheter nach Anspruch 4, wobei das Benetzungsmittel Polyvinylalkohol oder Heparin ist oder wobei die Hülse vor dem Ausdehnen benetzt wird, den Transfer des therapeutischen Mittels extern zur Hülse im nicht ausgedehnten Zustand jedoch im Wesentlichen beschränkt.

6. Ballonkatheter nach Anspruch 1, wobei die Hülse in einem Behälter eine mikroskopische Benetzung durchläuft, während Ballon und Hülse sich im nicht ausgedehnten Zustand befinden und an eine gewünschte Position in einem Gefäß geliefert werden.

7. Ballonkatheter nach Anspruch 1, wobei Körperflüssigkeiten die Hülse im Wesentlichen benetzen, wenn die Hülse ausgedehnt ist.

8. Ballonkatheter nach Anspruch 7, wobei die Beschichtung die Hülse auch benetzt, wenn die Hülse ausgedehnt ist, oder wobei bis zu dem Zeitpunkt, wenn die Hülse vollständig ausgedehnt ist, im Wesentlichen die gesamte Hülse nass ist, oder wobei das Benetzen der Hülse erleichtert wird, wenn sich die Hülse in Kontakt zu einer Gefäßwand befindet.

9. Ballonkatheter nach Anspruch 1, wobei die Hülse ein Fluorpolymer umfasst, optional wobei das Fluorpolymer ePTFE ist.

10. Ballonkatheter nach Anspruch 1, wobei die Knoten in Längsrichtung zur Längsachse des Ballonkatheters ausgerichtet sind und die Fibrillen in Umfangsrichtung zu der Achse ausgerichtet sind, optional wobei der Abstand zwischen den Knoten beim Ausdehnen der Hülse zunimmt.

11. Ballonkatheter nach Anspruch 1, wobei die Knoten in Umfangsrichtung zur Längsachse des Ballonkatheters ausgerichtet sind und die Fibrillen in Längsrichtung zu der Achse ausgerichtet sind, optional wobei der Abstand zwischen den Fibrillen beim Ausdehnen der äußeren Hülse zunimmt oder die Fibrillen sich beim Ausdehnen der äußeren Hülse neu ausrichten.

12. Ballonkatheter nach Anspruch 1, wobei die Beschichtung eine hydrophile Komponente umfasst.

13. Ballonkatheter nach Anspruch 12, wobei die hydrophile Komponente den Löslichkeitspunkt eines hydrophoben therapeutischen Mittels anhebt oder wobei die Beschichtung mindestens eine Komponente ausgewählt aus der folgenden Gruppe umfasst: Benzethoniumchlorid, PEG, Poloxamer, Natriumsalicylat und Hydroxypropyl-β-cyclodextrin.

14. Ballonkatheter nach Anspruch 1, wobei das therapeutische Mittel ein hydrophiles Mittel ist oder wobei das therapeutische Mittel ein hydrophobes Mittel ist; optional wobei das hydrophobe Mittel ausgewählt ist aus der Gruppe, bestehend aus taxandomänenbindenden Arzneimitteln, wie beispielsweise Paclitaxel und Rapamycin.

15. Ballonkatheter nach Anspruch 1, wobei der Ballonkatheter ferner eine Strukturschicht umfasst, optional wobei die Strukturschicht die Beschichtung und das therapeutische Mittel umfasst; oder wobei sich die Mikrostruktur der Hülse beim Ausdehnen des Ballons ändert.

## Revendications

1. Cathéter à ballonnet comprenant :
a. un ballonnet comprenant un revêtement et un agent thérapeutique sur une surface externe dudit ballonnet ;
b. une gaine disposée autour dudit ballonnet, ladite gaine présentant une microstructure composée de noeuds interconnectés par des fibrilles et ladite gaine possédant une microstructure à perméance variable, à l'état non dilaté la gaine limitant sensiblement le transfert dudit agent thérapeutique ;
c. ledit revêtement et ledit agent thérapeutique étant disposés entre la surface du ballonnet et la gaine ; et
d. lorsque ladite gaine est dilatée par le ballonnet, ladite gaine permettant le transfert dudit revêtement et dudit agent thérapeutique à travers la microstructure perméable de la gaine.

2. Cathéter à ballonnet selon la revendication 1, ledit revêtement et ledit agent thérapeutique étant transférés vers une zone externe à ladite gaine.

3. Cathéter à ballonnet selon la revendication 2, ledit revêtement et ledit agent thérapeutique étant transférés à travers ladite gaine et sur un tissu cible, éventuellement ledit revêtement restant sensiblement collé au tissu cible après le dégonflage du ballonnet.

4. Cathéter à ballonnet selon la revendication 1, ladite gaine étant traitée avec un agent mouillant.

5. Cathéter à ballonnet selon la revendication 4, ledit agent mouillant étant de l'alcool polyvinylique ou de l'héparine, ou ladite gaine s'humidifiant avant dilatation mais limitant sensiblement le transfert dudit agent thérapeutique externe à ladite gaine à l'état non dilaté.

6. Cathéter à ballonnet selon la revendication 1, ladite gaine subissant un mouillage microscopique dans un vaisseau pendant que ledit ballonnet et ladite gaine sont à l'état non dilaté et sont en train d'être délivrés à un emplacement souhaité à l'intérieur du vaisseau.

7. Cathéter à ballonnet selon la revendication 1, les fluides corporels humidifiant sensiblement la gaine lorsque ladite gaine est dilatée.

8. Cathéter à ballonnet selon la revendication 7, ledit revêtement mouillant également la gaine lorsque ladite gaine est dilatée, ou sensiblement toute ladite gaine étant mouillée le temps que ladite gaine est complètement dilatée, ou ledit mouillage de la gaine étant facilité lorsque ladite gaine est en contact avec une paroi de vaisseau.

9. Cathéter à ballonnet selon la revendication 1, ladite gaine comprenant un polymère fluoré, éventuellement ledit polymère fluoré étant du PTFEe.

10. Cathéter à ballonnet selon la revendication 1, lesdits noeuds étant alignés longitudinalement à l'axe longitudinal dudit cathéter à ballonnet et lesdites fibrilles étant alignées circonférentiellement audit axe, éventuellement la distance entre lesdits noeuds augmentant pendant que ladite gaine se dilate.

11. Cathéter à ballonnet selon la revendication 1, lesdits noeuds étant alignés circonférentiellement à l'axe longitudinal dudit cathéter à ballonnet et lesdites fibrilles étant alignées longitudinalement audit axe ; éventuellement la distance entre lesdites fibrilles augmentant pendant que la gaine externe se dilate, ou lesdites fibrilles se réorientant pendant que ladite gaine externe se dilate.

12. Cathéter à ballonnet selon la revendication 1, ledit revêtement comprenant un composant hydrophile.

13. Cathéter à ballonnet selon la revendication 12, ledit composant hydrophile augmentant le point de solubilité d'un agent thérapeutique hydrophobe, ou ledit revêtement comprenant au moins un composant choisi dans le groupe suivant : le chlorure de benzéthonium, le PEG, le poloxamère, le salicylate de sodium et l'hydroxypropyl-β-cyclodextrine.

14. Cathéter à ballonnet selon la revendication 1, ledit agent thérapeutique étant un agent hydrophile, ou ledit agent thérapeutique étant un agent hydrophobe ; éventuellement ledit agent hydrophobe étant choisi dans le groupe constitué par les médicaments de liaison au domaine taxane, tels que le paclitaxel, et la rapamycine.

15. Cathéter à ballonnet selon la revendication 1, ledit ballonnet comprenant en outre une couche structurelle, éventuellement ladite couche structurelle comprenant ledit revêtement et ledit agent thérapeutique ; ou ladite microstructure de la gaine se modifiant pendant que ledit ballonnet se dilate.
